# EUROPEAN PATENT APPLICATION

(11) **EP 1 723 962 A1**
(43) Date of publication of application: **22.11.2006**
(21) Application number: 05450090.5
(22) Date of filing: 19.05.2005
(51) Int. Cl.: A61K 38/48

(54) **Use of inhibitors of the renin-angiotensin system for the treatment of lung injuries**

(71) Applicant: IMBA-Institut für Molekulare Biotechnologie GmbH, 1030 Vienna (AT)
(72) Inventor: Penninger, Josef, 1130 Wien (AT); Imai, Yumico, 1070 Wien (AT); Kuba, Keiji, 1070 Wien (AT)
(74) Representative: Sonn & Partner Patentanwälte

(57) **Abstract**

The invention relates to the use of Angiotensin converting enzyme 2 (ACE2) for the preparation of a medicament for the treatment of severe acute lung injury, especially induced by acid aspiration or sepsis, of lung oedemas and lung injuries and failures connected with infection with severe acute respiratory syndrome (SARS) coronavirus.

## Description

The present invention relates to methods of treatment of lung injuries and lung diseases.

During several months of 2003, a newly identified illness termed severe acute respiratory syndrome (SARS) spread rapidly through the world disrupting travel, economics, and even scientific conventions. A novel coronavirus was identified as the SARS pathogen which triggered atypical pneumonia characterized by high fever and severe dyspnea. The death rate following infection approached almost 10% due to the development of acute severe lung failure. Moreover, influenza such as the Spanish flu and the emergence of new respiratory disease viruses have caused high lethality among infected individuals due to acute lung failure.

The high lethality of SARS infections, its enormous economic and social impact, fears of renewed outbreaks of SARS as well as the feared misuse of such viruses as biologic weapons make it paramount to understand the disease pathogenesis of SARS and acute severe lung failure. Recently, Angiotensin Converting Enzyme 2 (ACE2) was identified as a functional SARS-coronavirus receptor in cell lines (Li et al., 2003; Wang et al., 2004). However, a possible second receptor, CD209L (L-SIGN) has been identified in in vitro cell line studies (Jeffers, et al., 2000). Thus, it was not known whether ACE2 is indeed a critical component involved in SARS infections in vivo.

Acute respiratory distress syndrome (ARDS), the most severe form of acute lung injury, is a devastating clinical syndrome with high mortality rate (30-60%). Predisposing factors for ARDS are diverse and include sepsis, aspiration, and pneumonias including infections with SARS coronavirus. To date no effective drugs are approved to improve clinical outcome of ARDS.

ACE2 is an angiotensin converting enzyme acting as a carboxypeptidase and cleaving a single residue from AngI, thereby generating Ang1-9, and a single residue from AngII to generate Ang1-7. Recombinant ACE2 (rACE2), methods of production and use of ACE2 are described i.a. in Crackower et al., 2002, WO 00/18899 A2, WO 02/12471 A2 or WO 2004/000367 A1.

It is an object of the present invention to provide efficient prophylactic ant therapeutic methods for combatting acute lung failures, especially connected with acid aspiration or sepsis, lung oedemas, ARDS and lung failures being connected with SARS virus infection. Furthermore, novel strategies for lung injuries and failures are needed.

Therefore the present invention provides the use of Angiotensin converting enzyme 2 (ACE2) for the preparation of a medicament for the treatment of severe acute lung injury, especially induced by acid aspiration or sepsis, of lung oedemas and lung injuries and failures connected with infection with severe acute respiratory syndrome (SARS) coronavirus.

Indeed, although it was known that ACE2 binds SARS proteins, it was found in the course of the present invention that the binding of SARS virus proteins (Spike) to ACE2 is responsible for the deleterious lung effects of SARS infection. These findings enabled a completely new line of combatting lung injuries and failures which are connected with SARS virus infection and opened up also new strategies for treatment of this infection. Moreover, since severe acute lung injuries or lung oedemas show (in contrast to other lung injuries and lung failures) similarity with these observations, specifically with respect to ACE2, these findings also opened up the possibility to effectively address severe acute lung injuries (specifically those induced by acid aspiration or sepsis) and lung oedemas. Generally, all patients with acute lung disorders (specifically all acute lung disorders which need intensive treatment, such as the ones described above or others, such as ARDS in general, Pneumonia-induced or Anthrax-induced acute lung injuries) which require treatment in the intensive health care unit of a hospital can benefit from ACE2 administration (alone or in combination with the specific inhibitors described below) according to the present invention, i.e. by externally substituting the loss of ACE2 being connected by these acute failures.

Preferably, the medicament according to the present invention comprises recombinant ACE2 (rACE2). rACE2 is reproducibly manufacturable even in large quantities. Variations from lot to lot and place of manufacture are only minor if GMP is applied. On the other hand also ACE2 mutants may be used as ACE2 component according to the present invention instead of the ACE2 protein with the natural amino acid sequence. Suitable mutants are described i.a. in WO 00/18899 A2, WO 02/12471 A2 or WO 2004/000367 A1.

On the other hand, according to the experimental work carried out for the present invention ACE2 and one or more inhibitors of the RAS (RAS-inhibitors) or bradykinin receptor inhibitors enable a synergisitc improvement of the action of ACE2 in lung disorders, preferably one or more of the following components (all these substances include pharmaceutically acceptable administration forms, such as salts, esters, depot forms, etc.): AT₁₋inhibitors (=selective blockers of the AT₁ receptor; often also referred to as "ANG II agonists" (US 2003/0171415)), AT₂-agonists (=selective effectors of the AT₂ receptor), ACE inhibitors, renin inhibitors and bradykinin receptor inhibitors. The combination of e.g. AT₁-inhibitor + ACE2, AT₂-agonist + ACE2, AT₁-inhibitor + AT₂-agonist + ACE2, ACE inhibitor + ACE2, renin inhibitor + ACE2 and bradykinin receptor inhibitor + ACE2 as well as combinations of AT₁-inhibitor (or: AT₂-agonist) + ACE inhibitor + ACE2, AT₁-inhibitor (or: AT₂-agonist) + bradykinin receptor inhibitor + ACE2, ACE inhibitor + bradykinin receptor inhibitor + ACE2, AT₁-inhibitor + ACE inhibitor + bradykinin receptor inhibitor + ACE2 are therefore examples for improved combination medicaments which show significant additional benefit for treatment and prophylaxis of various lung diseases, especially those having a connection to the renin-angiotensin system. These combination medicaments are specifically suited to treat or prevent severe acute lung injury (especially those induced by acid aspiration or sepsis), of lung oedemas and lung injuries and failures connected with infection with severe acute respiratory syndrome (SARS) coronavirus, especially lung oedemas.

Instead of using ACE2 as a protein (especially a recombinant protein) the medicament according to the present invention may also comprise a nucleic acid, especially a DNA molecule, encoding ACE2 in addition or instead of ACE2. ACE2 may then be delivered by nucleic acid shuttles (comprising a coding region for ACE2 and regulatory sequences for ACE2 expression in a vector). The inhibitor components of a combination drug can then be delivered together with the ACE2 gene shuttle, however, separate administration of the inhibitor component is preferred.

ACE2 DNA molecules may be administered, preferably in recombinant form, as plasmids, directly or as part of a recombinant virus or bacterium. Examples of in vivo administration are the direct injection of "naked" DNA, either by intramuscular route or using a gene gun. Examples of recombinant organisms are vaccinia virus, adenovirus or listeria monocytogenes (a summary was provided by Coulie, P.G. (1997), Mol. Med. Today 3, 261-268). Moreover, synthetic carriers for nucleic acids such as cationic lipids, microspheres, micropellets or liposomes may be used for in vivo administration of nucleic acid molecules coding for ACE2. The application may optionally be combined with a physical method, e.g. electroporation.

In principle, any method of gene therapy may be used; gene therapy systems useful in respiratory diseases are described in Klink et al., J Cyst Fibros. 2004 Aug;3 Suppl 2:203-12.

The present invention therefore encompasses also the use of a combination of ACE2 and an AT₁-inhibitor for the preparation of a medicament for the treatment of lung injuries or lung diseases.

Although the exact nature of the RAS-inhibitors, especially AT₁₋inhibitor, AT₂-agonists, renin inhibitors, ACE inhibitor, or bradykinin receptor inhibitor to be used according to the present invention is not critical and although there exists a large amount of inhibitor substances (which makes each of these inhibitors a generic group of substances), it is preferred to use those substances according to the present invention for which a clear (pharmacological) record of action, performance, toxicity, etc. (see e.g. "Guide to Receptors and Channels", especially "Angiotensin", "Bradykinin") Brit.J.Pharmacol.141, Suppl.1 (2004)) is present in order to enable a drug registration within rather short time frames. Therefore, the medicament according to the present invention preferably comprises an AT₁-inhibitor selected from the group consisting of candesartan, eprosartan, irbesartan, losartan, telmisartan, valsartan, olmesartan, tasosartan, embusartan, forsartan, milfasartan, pratosartan, ripisartan, saprisartan, zolasartan or mixtures thereof or a pharmaceutically acceptable salt thereof, especially telmisartan. A particular preferred AT₁-inhibitor is telmisartan or a pharmaceutically acceptable salt thereof.

According to a preferred embodiment, the present medicament comprises an ACE inhibitor, especially an ACE inhibitor selected from the group consisting of benazepril, captopril, ceronapril, enalapril, fosinopril, imidapril, lisinopril, moexipril, quinapril, ramipril, trandolapril, perindopril, alacepril, cilazapril, delapril, spirapril, temocapril, zofenopril or mixtures thereof, or a pharmaceutically acceptable salt thereof.

According to a preferred embodiment, the present medicament comprises a renin inhibitor, especially a renin inhibitor selected from the group consisting of [alpha-R[alpha-R*,beta-S*(S*,S*)]-alpha-hydroxy-beta-[[2-[[2-(4-morpholin- 1-carboxamido)-1-oxo-3-phenylpropyl]amino]-3-methylthio-1-oxo-propyl]amino] cyclohexanebutanoic acid, isopropyl ester; alisk(i)ren, zankiren, 2(S),4 (S),5(S),7(S)-N-(3-amino-2,2-dimethyl-3-oxopropyl)-2,7-di(1-methylet hyl)-4-hydroxy-5-amino-8-[4-methoxy-3-(3-methoxy-propoxy)phenyl]-octanamide, remikiren, or mixtures thereof, or a pharmaceutically acceptable salt thereof.

According to a further preferred embodiment, the present medicament comprises a bradykinin receptor inhibitor, preferably a des-Arg⁹-bradykinin-inhibitor, especially Lys-Lys [Hyp³,Cpg⁵,dTic⁷,Cpg⁸]des-Arg⁹]-bradykinin (B9958), AcLys-Lys ([αMe] Phe⁵,D-βNal⁷,Ile⁸]des-Arg⁹-bradykinin (R914), AcLys[D Nal⁷,Ile⁸] [des-Arg⁹]-bradykinin(R715), Lys-[Leu⁸] [des-Arg⁹]-bradykinin, DArg [Hyp³,Thi⁵,DTic⁷,Oic⁸]-bradykinin (icatibant; HOE140), 1-([2,4-dichloro-3-{([2,4-dimethylquinolin-8-yl]oxy)methyl}phenyl]sulphonyl)-N-(3-[{4-(aminomethyl)phenyl}carbonylamino)propyl)-2(S)-pyrrolidinecarboxamide (anatibant; LF160687), (E)-3-(6-acetamido-3-pyridyl)-N-(N-[2,4-dichloro-3{(2-methyl-8-quinolinyl)oxymethyl}phenyl]-N-methylaminocarbonyl-methyl)acrylamide (FR173657), [[4-[[2-[[bis(cyclohexylamino)methylene] amino]-3-(2-naphthyl)-1-oxopropyl]amino]phenyl]methyl]tributylphosphonium chloride monohydrochloride (WIN 64338), bradyzyte (British Journal of Pharmacology (2000) 129, 77 - 86), (S)-1-[4-(4-benzhydrylthiosemicarbazido)-3-nitrobenzenesulfonyl]pyrrolidine-2-carboxylic acid [2-[(2-dimethylaminoethyl)methylamino]ethyl] amide (bradyzide; (S)-4), bradykinin B(2) receptor antagonists described in Curr Med Chem. 2002 May;9(9):913-28, or mixtures thereof, or a pharmaceutically acceptable salt thereof.

According to the present invention it is also possible to use bradykinin receptor inhibitors without ACE2, AT₁-inhibitor or ACE inhibitor for the treatments envisaged by the present invention. The present invention therefore also relates to the use of the bradykinin receptor inhibitors for lung injuries or lung diseases, especially severe acute lung injury induced by acid aspiration or sepsis, of lung oedemas and lung injuries and failures connected with infection with severe acute respiratory syndrome (SARS) coronavirus. A specifically preferred aspect of the present invention is the use of bradykinin receptor inhibitors (of BK1 and/or BK2) for the preparation of a medicament for the treatment of lung oedemas. This lung oedema medicament comprising a bradykinin receptor inhibitor may also be combined with ACE2 or other RAS-inhibitors as described herein.

Although administration of the combination according to the present invention may be performed by separate administration, combined administration as combination medicament is preferred (if ACE2 is administered in protein form).

The (combination) medicament according to the present invention is preferably administered intravenously, intraperitoneally, mucosally, especially intranasally, orally or intratracheally, or as an aerosol composition. The relative amounts of the different components are within the typical administration doses or may be below individual doses of the single medicament (because of the synergisitc effects).

The present invention also relates to combination medicaments comprising ACE2 and one or more RAS inhibitors and/or bradykinin receptor inhibitor, especially bradykinin receptor inhibitor, AT₁-inhibitor, renin inhibitor, AT₂-agonist and ACE inhibitor.

The present invention therefore also encompasses a combination medicament e.g. selected from the group consisting of AT₁-inhibitor + ACE2, AT₂-agonist + ACE2, AT₁-inhibitor + AT₂-agonist + ACE2, ACE inhibitor + ACE2, renin inhibitor + ACE2 and bradykinin receptor inhibitor + ACE2 as well as combinations of AT₁₋inhibitor (or: AT₂-agonist) + ACE inhibitor + ACE2, AT₁-inhibitor (or: AT₂-agonist) + bradykinin receptor inhibitor + ACE2, ACE inhibitor + bradykinin receptor inhibitor + ACE2, AT₁-inhibitor + ACE inhibitor + bradykinin receptor inhibitor + ACE2. These combination medicaments may preferably be mixed with a suitable pharmaceutically acceptable carrier, diluent or excipient. ACE2 and the inhibitors or ACE2/inhibitor combinations listed above may also be combined with further RAS-inhibiting substances (US 6,387,894), such as renin inhibitors (e.g those described in Pharm. Res., 4, 364-374 (1987), US 4,814,342, 4,855,303, 4,895,834 and US 6,387,894), AT₂-receptor (AT₂), AT₂-activators (e.g. p-amino-Phe⁶ angiotensin II (p-NH2Phe6-Ang II), Nic-Tyr-(epsilon-CBZ (benzyloxycarbonyl)-Arg)Lys-His- Pro-Ile (CGP42112), those described in US 6,762,167, US 6,747,008 or US 6,444,646)); etc..

Since the control of lung oedema formation by RAS is another surprising fact provided by the present invention, another aspect of the present invention is the use of an inhibitor of the Renin-Angiotensin-System for the prevention or treatment of lung injuries or lung failures, preferably of severe acute lung injury induced by acid aspiration or sepsis, of lung oedemas and lung injuries and failures connected with infection with SARS coronavirus, especially of lung oedemas. The factors that may trigger lung oedemas are rather diverse (and in general all of them can be addressed by the present invention), the preferred causes of lung oedemas according to the present invention are - besides sepsis, acid aspiration or SARS - influenza, anthrax (bioterrorism), bacteria, fungi, pancreatitis, near drowning, acute poisons etc.. The agents and combination medicaments of the present invention are also useful for the preparation of a drug for the treatment of pathologically enhanced vascular permeability in the lung. A specifically preferred aspect of the present invention is the use of an RAS-inhibitor for the preparation of a medicament for the treatment of lung oedemas. The RAS inhibitors may be applied individually or, preferably (due to the synergistic effects), as combination medicaments of two or more different RAS-inhibitors, especially the at least two RAS-inhibitors having also different targets.

Preferred inhibitors of the Renin-Angiotensin-System are selected from the group consisting of ACE inhibitors, ACE2, AT₁-inhibitors, AT₂-receptor, AT₂-activators, renin inhibitors or combinations thereof.

Since the level of ACE2 in the specific lung diseases described in the present invention correlates to the status and progression of these diseases, a further aspect of the present invention is the use of ACE2 and serum or lung Angiotensin II levels as a disease marker for severe acute lung injuries (especially those induced by acid aspiration or sepsis), of lung oedemas and lung injuries and failures connected with infection with severe acute respiratory syndrome (SARS) coronavirus. The ACE2 levels and Angiotensin II (AngII) levels of samples taken from patients (especially from serum or lung) may therefore be used to correlate ACE2 or AngII levels to progression of disease and success of treatment measures, preferably in comparison with previous samples or standard ACE2 or AngII values (e.g. of standard normal levels or values from healthy individuals). Such diagnostic measurements of ACE2 or AngII are in general known to the skilled man in the art; e.g. the methods described in WO 00/18899 A2, WO 02/12471 A2 or WO 2004/000367 A1 may easily be adapted to the present invention.

The present invention is further described by the following examples and the drawing figures, yet without being restricted thereto.

### Figures:

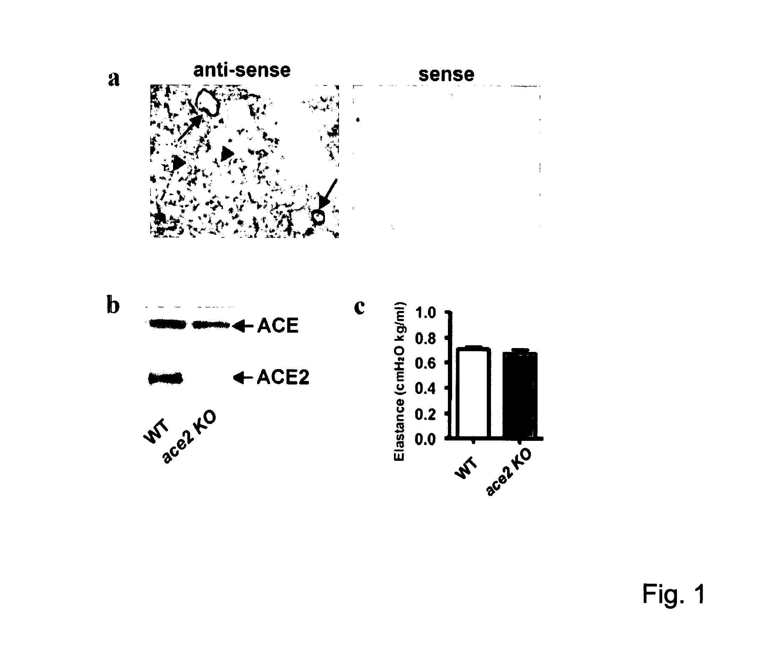

Figure 1. ACE2 expression in mouse lungs a, In situ hybridization analysis of ACE2 mRNA expression. Lung sections from wild-type mice were hybridized with digoxygenenin-labeled anti-sense and sense RNA probes spanning ACE2 exons 11 to 18 (not homologous to ACE sequence). b, Western blot analysis of ACE2 and ACE expression in lung tissue extracts from wild-type (WT) and ace2 KO mice. c, Basal lung elastance of WT and ace2 KO mice. n = 4-6 per group. Data are shown as mean +/s.e.m..;

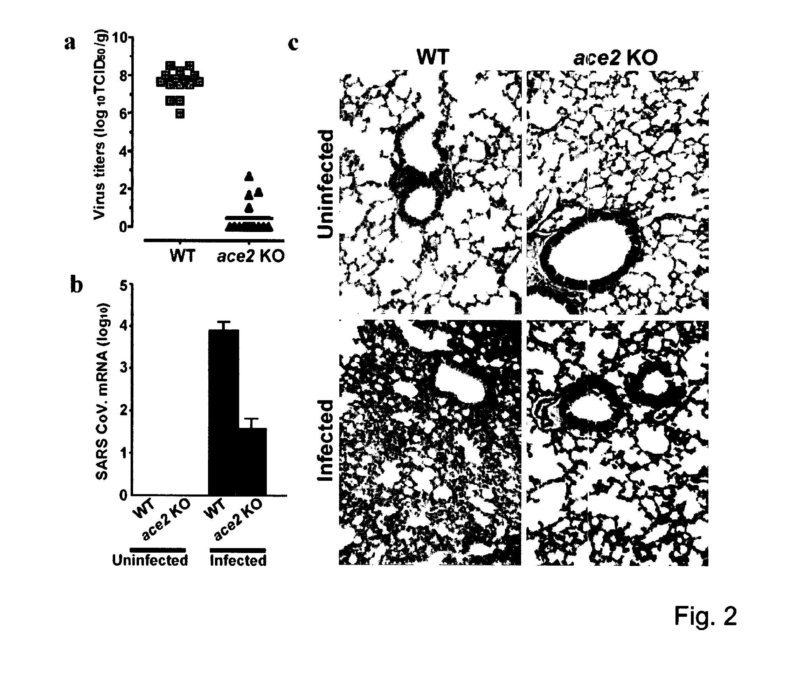

Figure 2. ACE2 is a critical receptor for SARS infections in vivo: a,b SARS coronavirus replication (a) and detection of SARS mRNA (b) in wild type (WT) and ace2 KO mice. Mice were infected with the Beijing strain of SARS-CoV (isolate PUMC01 AY350750, 105.23 virus; Zou, K et al., 2003). Viral replication was determined from lung tissue at day 2 of infection (see Methods). Virus titers (mean log₁₀TCID₅₀ per g lung tissue) are shown on a log₁₀ scale for individual mice. n = 15 per group. SARS mRNA expression was assayed using real-time RT-PCR of SARS Spike. Spike RNA copy numbers were normalized by mouse β-actin copy numbers at each sample. Data is shown as mean values +/- s.e.m. on a log10 scale. n = 15 per group. c, Lung histopathology of control and SARS infected WT and ace2 KO mice. Lungs were taken on day 8 after SARS infection. Note leukocyte infiltration in SARS infected WT mice while none of the 15 analyzed ace2 KO mice showed any signs of lung pathologies or lung inflammation. An image of a severe WT case is shown (H&E, x 200).

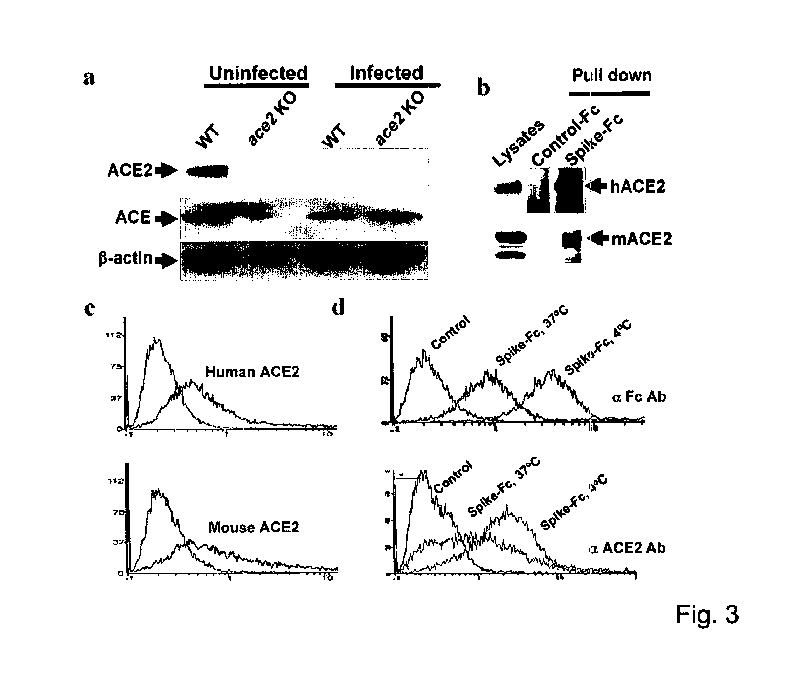

Figure 3. Downregulation of ACE2 expression by SARS infection and recombinant SARS Spike protein: a, Decreased ACE2 protein, but normal ACE levels, in the lung of mice after SARS infection. Lung homogenates were prepared from control and SARS-infected wild type or ace2 KO mice and analyzed by Western Blot. b, Binding of recombinant Spike(S-1190)-Fc protein (S-1190) to human ACE2 (hACE2) and mouse ACE2 (mACE2) in pull-down assay. Spike-Fc but not control Fc protein pulled down hACE2 and mACE2 from total cell extracts of A549 human alveolar epithelial cells and IMCD murine kidney epithelial cells, respectively. Total lysates are shown as controls. c, Binding of Spike-Fc protein to hACE2 and mACE2 in cell culture. hACE2- or mACE2-transfected 293 cells are incubated with Spike-Fc and the binding was detected by FACS (blue lines). Non-transfected 293 cells incubated with Spike-Fc followed by anti-Fc Abs are shown as controls (black line) d, Decreased cell surface expression of ACE2 following binding to Spike-Fc protein at 37°C but not 4°C in Vero E6 cells. ACE2 surface expression was detected at 3 hours of incubation with Spike-Fc using an anti-ACE2 monoclonal Ab. Similar data were obtained using anti-Fc Ab to directly detect surface bound Spike-Fc and to avoid masking of the ACE2 epitope. Representative FACS histograms are shown including a background control with an isotype matched Ab.

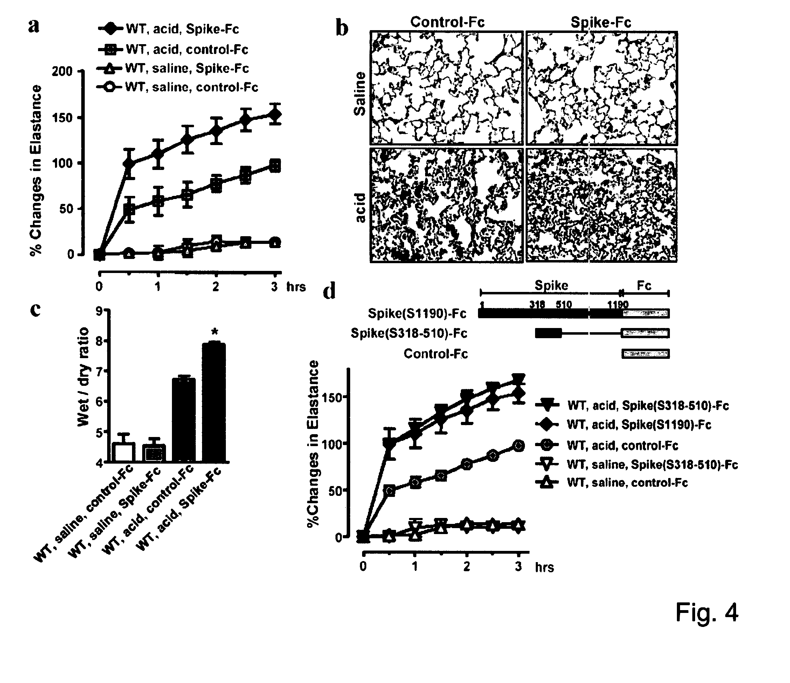

Figure 4. The SARS Spike protein enhances the severity of severe acute lung injury: a, Lung elastance measurements after saline or acid instillation in Spike-Fc protein- or control-Fc-treated WT mice. n = 5-7 per group. p < 0.05 for the whole time course comparing Spike-Fc-treated and control-Fc-treated WT mice after acid injury (repeated ANOVA test). b, Lung histopathology. Note enhanced alveolar wall thickening leukocyte infiltration, lung oedema, and partial destruction of alveolar structures in Spike-Fc treated WT mice. Representative images are shown (H&E, x 200). c, Wet to dry weight ratios of lungs as read-out for pulmonary oedema in control and Spike-Fc-treated mice in the presence or absence of acid-induced lung injury. Bars show mean values +/- s.e.m. Asterisk, p < 0.05 between control and Spike-Fc-treated mice with acid challenge (ANOVA and two-tailed t-test). d, Severe acute lung failure by Spike(S318-510)-Fc treatment in acid-challenged mice. The scheme (upper panel) shows the ACE2-binding domain of Spike protein (S318-510). Lung elastance measurements (lower panel) showed that Spike(S318-510)-Fc induced severe acute lung failure in acid-challenged WT mice, comparable to Spike(S1190)-Fc. n = 5-7 per group. p < 0.05 for the whole time course comparing Spike(S318-510)-Fc or Spike(S1190)-Fc-treated and control-Fc-treated WT mice after acid injury (repeated ANOVA test).

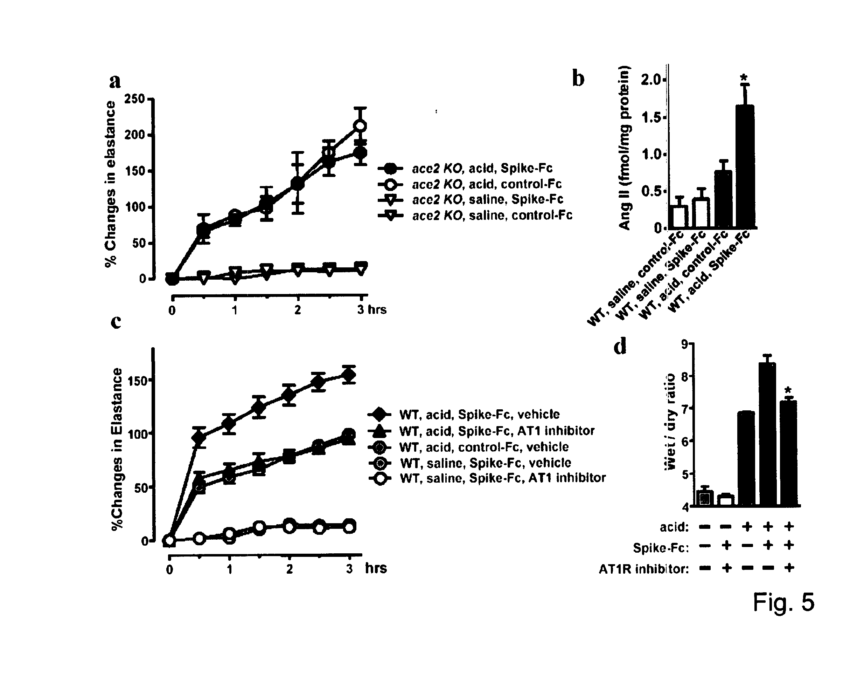

Figure 5. Inhibition of the angiotensin II receptor (ATIR) attenuates SARS Spike-mediated severe acute lung injury: a, Lung elastance measurements after acid instillation in Spike-Fc protein (S1190) - or control-Fc-treated ace2 KO mice. n = 5-7 for each group. There were no significant differences between Spike-Fc-treated ace2 KO and control-Fc-treated ace2 KO mice after acid injury. b, Ang II peptide levels in lungs of Spike-Fc protein- or control-Fc-treated WT mice following saline or acid aspiration. AngII levels were determined at 3 hours by EIA. Bars show mean values ± s.e.m. Asterisk, p < 0.05 comparing Spike-Fc- and control-Fc-treated WT mice after acid injury. c, Lung elastance measurements in acid plus Spike-Fc challenged WT mice treated with the AT1R inhibitor Losartan (see Methods). n = 4-6 per group. p < 0.05 comparing AT1R inhibitor-treated Spike-Fc challenged mice with vehicle-treated Spike-Fc challenged mice (ANOVA and two-tailed t-test). d, Wet to dry weight ratios of lungs of acid and Spike-challenged mice in the presence or absence of the specific AT1R blocker Losartan. n = 4-6 mice per group. Bars show mean values +/- s.e.m. Asterisk, p < 0.05, comparing AT1R inhibitor-treated ace2 KO with vehicle-treated ace2 KO mice at 3 hrs after acid injury (ANOVA and two-tailed t-test).

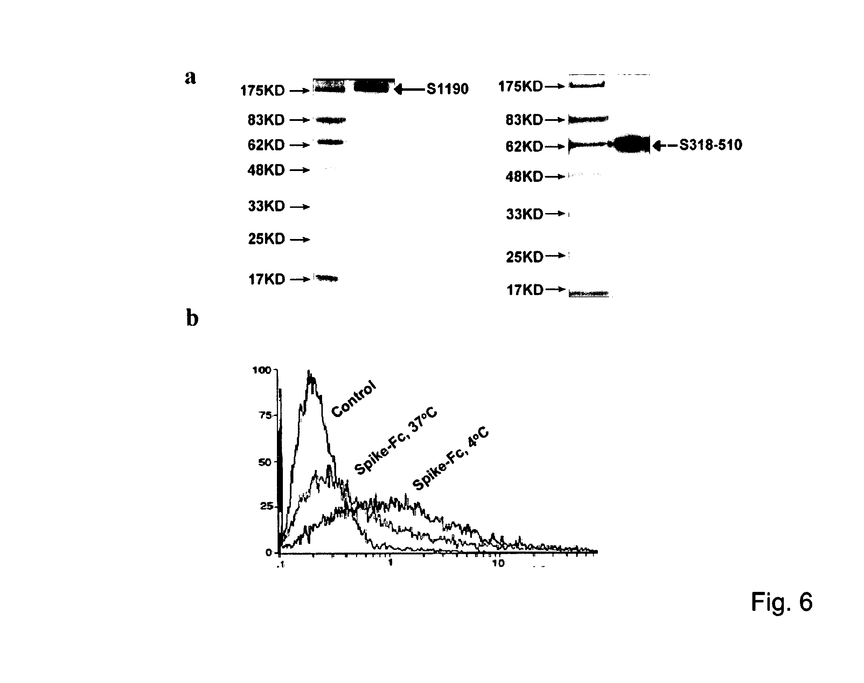

Figure 6. Recombinant Spike-Fc proteins and reduced ACE2 expression by Spike (S318-510)-Fc: a, Commassie-stained SDS-PAGE gels of purified recombinant Spike(S1190)-Fc and Spike(S318-510)-Fc proteins isolated from the culture supernatants of transfected CHO cells. Molecular weights are indicated. b, Decreased cell surface expression of ACE2 following binding to Spike (S318-510)-Fc protein at 37°C but not 4°C in Vero E6 cells. ACE2 surface expression was detected at 3 hours of incubation with Spike (318-510)-Fc using an anti-ACE2 monoclonal Ab. Representative FACS analysis is shown including a background control with an isotype matched Ab.

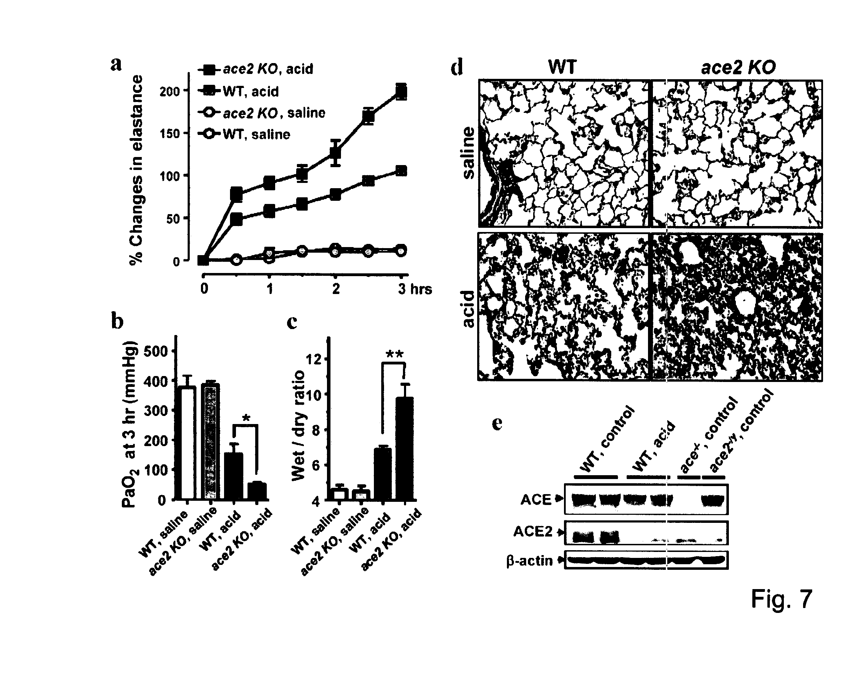

Figure 7. Loss of ACE2 worsens acid aspiration-induced acute lung injury: a, Lung elastance measurements after acid or saline treatment in wild type (WT) and ace2 knockout (ace2 KO) mice. n = 10 for each acid treated group. n = 6 for each saline control group. Asterisk, p < 0.05 for the whole time course comparing acid-treated WT and ace2 KO mice (repeated measurement ANOVA). b, Partial pressure of oxygen in arterial blood (PaO₂) from WT and ace2 KO mice in acid-induced ALI. c, Wet to dry weight ratios of lungs as read-out for pulmonary oedema in control and acid-treated WT and ace2 KO mice 3hrs after acid injury. Bars in b and c show mean values +/- s.e.m. Asterisk, p < 0.05; double asterisk, p < 0.01 between acid-treated WT and ace2 KO mice (ANOVA and two-tailed t-test). d, Lung histopathology. Note enhanced hyaline membranes formation, inflammatory cell infiltration, and lung oedema in acid treated ace2 KO mice. Note the normal lung morphology in saline treated WT and ace2 KO controls. Representative images are shown (H&E, x 200). e, ACE2 and ACE protein expression in control lungs and lungs at 3 hrs after acid injury. Total lung homogenates were analysed by Western blotting. β-actin is shown as a loading control. One experiment representative of 5 different experiments is shown.

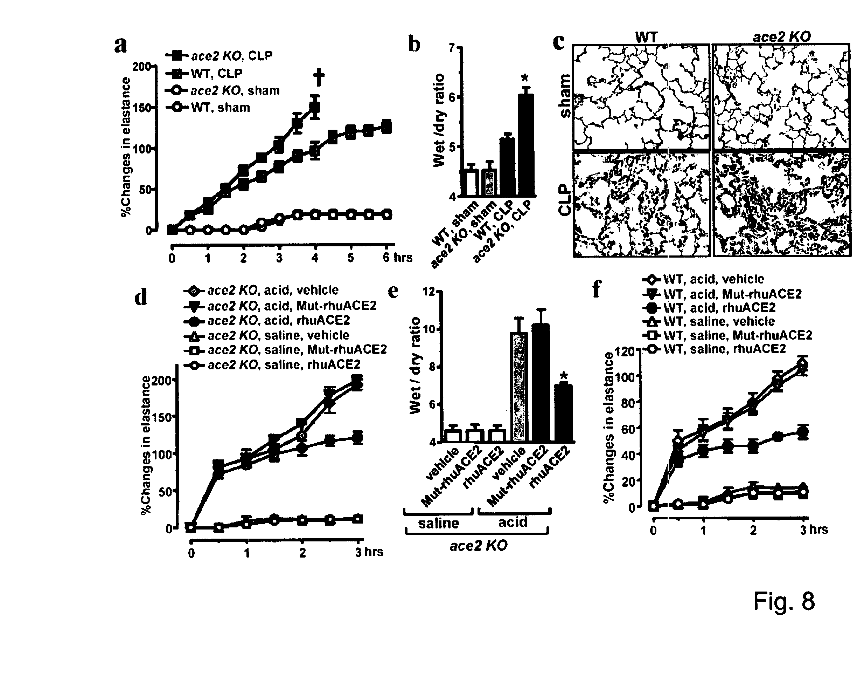

Figure 8. Loss of ACE2 worsens sepsis-induced ALI and rhuACE2 protein reduces the severity of ALI in ace2 mutant and wild type mice: a, Lung elastance measurements after sepsis-induced ALI in WT and ace2 KO mice. 18 hrs after sham or CLP surgery, animals received mechanical ventilation for another 6 hrs. n = 10 each in CLP-treated groups. n = 6 each in sham-treated controls. Since 8 out of 10 CLP-treated ace2 KO mice died between 4 and 4.5 hrs, only data up to 4 hours are shown for the ace2 KO group. Lung elastance was significantly higher in ace2 KO than WT mice at 4 hrs. p < 0.01 comparing CLP-treated ace2 KO mice with CLP-treated WT mice (ANOVA and two-tailed t-test). b, Wet to dry weight ratios of lungs in sham and CLP-treated WT and ace2 KO mice were determined at 4 hrs of ventilation. Bars show mean values +/- s.e.m. Asterisk, p < 0.05 between CLP-treated WT and ace2 KO mice (ANOVA and two-tailed t-test). c, Lung histopathology in sham and CLP-treated WT and ace2 KO mice. Note enhanced lung oedema and inflammatory infiltrates in ace2 KO mice, compared with WT mice. Representative images at 4 hrs of ventilation are shown (H&E, x 200). d, Lung elastance after acid and control saline instillation of ace2 KO mice treated with recombinant human ACE2 protein (rhuACE2; 0.1 mg/kg), mutant rhuACE2 (Mut-rhuACE2; 0.1 mg/kg), or vehicle alone (see Methods) i.p.. n = 6 per group. Asterisk, p < 0.05 comparing rhuACE2-treated ace2 KO mice with Mut-rhuACE2-treated and vehicle-treated ace2 KO mice at 3 hours. (ANOVA and two-tailed t-test). e, Wet to dry weight ratios of lungs in rhuACE2- , Mut-rhuACE2-, or vehicle-treated ace2 KO mice in acid-induced ALI. Bars show mean values +/- s.e.m. Asterix, p < 0.05 comparing rhuACE2-treated with Mut-rhuACE2-treated or vehicle-treated ace2 KO mice at 3 hours. (ANOVA and two-tailed t-test). f, Lung elastance measurements after acid instillation in WT mice treated with rhuACE2 protein (0.1 mg/kg), Mut-rhuACE2 protein (0.1 mg/kg), or vehicle. n = 6-8 per group. Asterisk p < 0.05 comparing rhuACE2-treated with Mut-rhuACE2-treated or vehicle-treated WT mice at 3 hours (ANOVA and two-tailed t-test).

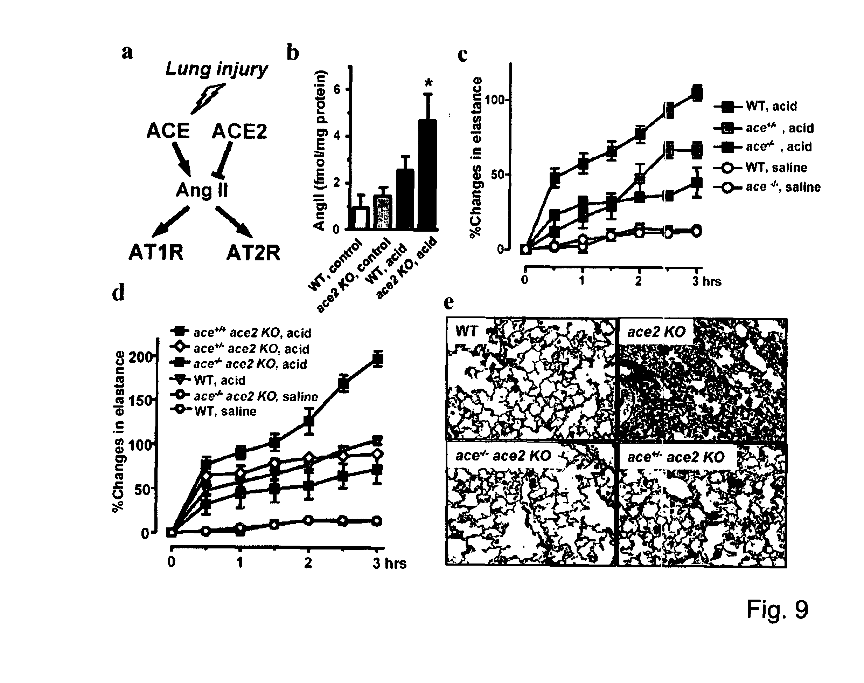

Figure 9. ACE deficiency reduces the severity of acute lung injury: a, Schematic diagram of the renin-angiotensin system. b, Lung AngII levels in control and acid-treated WT and ace2 KO mice. AngII levels were determined at 3 hrs by EIA. Bars show mean values +/- s.e.m. (n = 3-5 per group). Asterisk, p < 0.05 (ANOVA and two-tailed t-test) between acid treated WT and ace2 KO mice. c, Lung elastance measurements after acid instillation in ace^{+/+} (WT), ace^{+/-} and ace^{-/-} mice. n = 4-6 mice per group. Asterisk p < 0.05 comparing WT with ace^{+/-} and ace^{-/-} mice at 3 hrs (ANOVA and two-tailed t-test). Data from saline-treated ace^{-/-} and WT mice are shown as controls. d, Lung elastance measurements in acid-treated ace2 KO, ace^{+/-}ace2 KO, ace^{-/-}ace2 KO and WT mice and saline-treated ace^{-/-}ace2 KO and WT mice. n = 5 mice per group. Asterisk p < 0.05 comparing ace2 KO with WT, ace^{+/-}ace2 KO, or ace^{-/-}ace2 double mutant mice at 3 hrs after acid-treatment (ANOVA and two-tailed t-test). Without injury, there were no differences in lung elastance among the groups. e, Lung histopathology. Severe lung interstitial oedema and leukocyte infiltration in ace2 KO mice are attenuated by homozygosity (ace^{-/-}) or heterozygosity (ace^{+/-}) for the ace mutation. Representative micrographs are shown (H&E, x200).

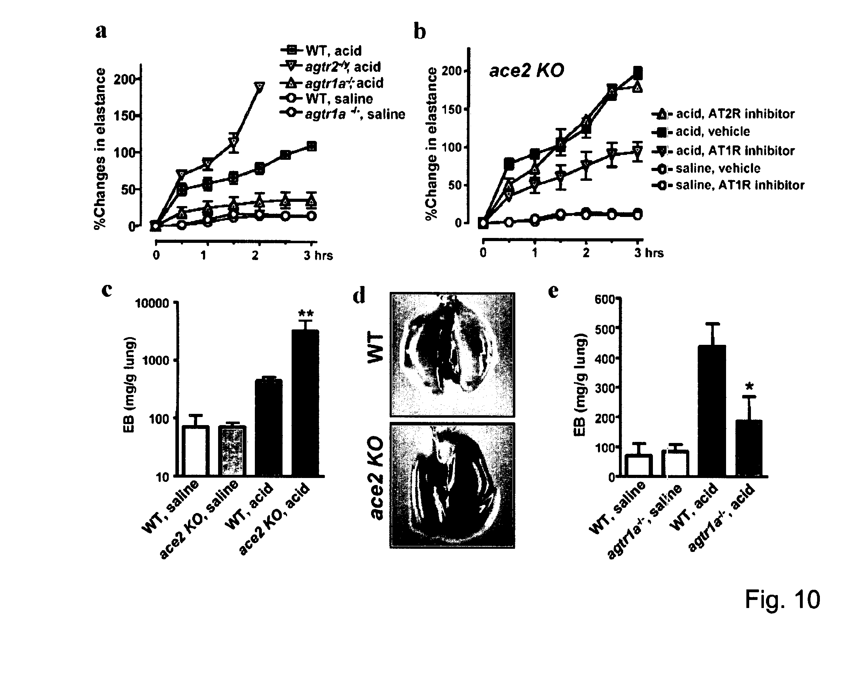

Figure 10. The AngII receptor ATlaR controls ALI severity and pulmonary vascular permeability: a, Lung elastance measurements in agtrla^{-/-} mice, agtr2^{-/y} mice, and WT mice after acid aspiration. n = 4-6 per group. All acid-treated agtr2^{-/y} mice died after 2 hrs. Double asterisk p < 0.01 for the whole time course comparing acid-treated WT and acid-treated agtrla^{-/-} mice (repeated ANOVA test). p < 0.01 comparing WT with agtr2^{-/y} mice at 2 hrs (ANOVA and two-tailed t-test). b, Lung elastance measurements in ace2 KO mice treated with AT1R (Losartan, 15 mg/kg) or AT2R (PD123.319, 15 mg/kg) inhibitors, or vehicle after acid or saline instillation (see Methods). n = 4-6 per group. Double asterisk p < 0.01 comparing AT1R inhibitor-treated ace2 KO with vehicle-treated or AT2R inhibitor-treated ace2 KO mice at 3 hrs (ANOVA and two-tailed t-test). c, Pulmonary vascular permeability as determined by intravenous injection of Evans Blue (EB). Extravascular EB in lungs was measured in WT and ace2 KO mice 3 hrs after acid injury (see Methods). Bars show mean values +/s.e.m. (n = 5 per group). Double asterix, p < 0.01 comparing acid-treated WT and ace2 KO mice at 3 hours (ANOVA and two-tailed t-test). d, Representative images of EB-injected lungs of WT and ace2 KO mice 3 hrs after acid-aspiration. e, Extravascular EB in lungs of WT and agtrla KO mice 3 hours after acid injury. Bars show mean values +/- s.e.m. (n = 5 per group). Asterix, p < 0.05 comparing acid-treated WT and agtrla KO mice at 3 hrs (ANOVA and two-tailed t-test).

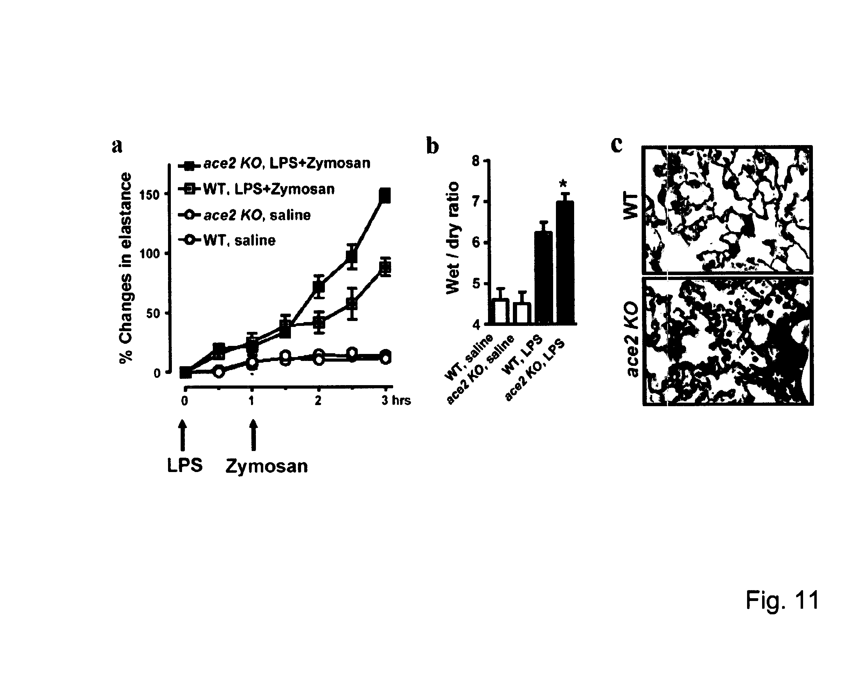

Figure 11. Severe endotoxin-induced lung injury in ace2 mutant mice: a, Lung elastance measurements after endotoxin-induced ALI in WT and ace2 KO mice. One hour after intratracheal instillation of LPS (0.5 µg/g), mice were challenged with zymosan (3 µg/g). n = 6 each in LPS+zymosan treated groups. n = 5 each in saline controls. p < 0.05 comparing acid-treated WT and ace2 KO mice at 3 hrs (ANOVA and two-tailed t-test). b, Wet to dry weight ratios of lungs in WT and ace2 KO mice in LPS+zymosan-induced acute lung injury. Bars show mean values +/- s.e.m. Asterisk, p < 0.05 between LPS+zymosan-treated WT and ace2 KO mice (ANOVA and two-tailed t-test). c, Lung histopathology in LPS+zymosan treated in WT and ace2 KO mice. Note enhanced lung oedema and inflammatory cell infiltrates in ace2 KO mice compared with WT mice. Representative images are shown (H&E, x 400).

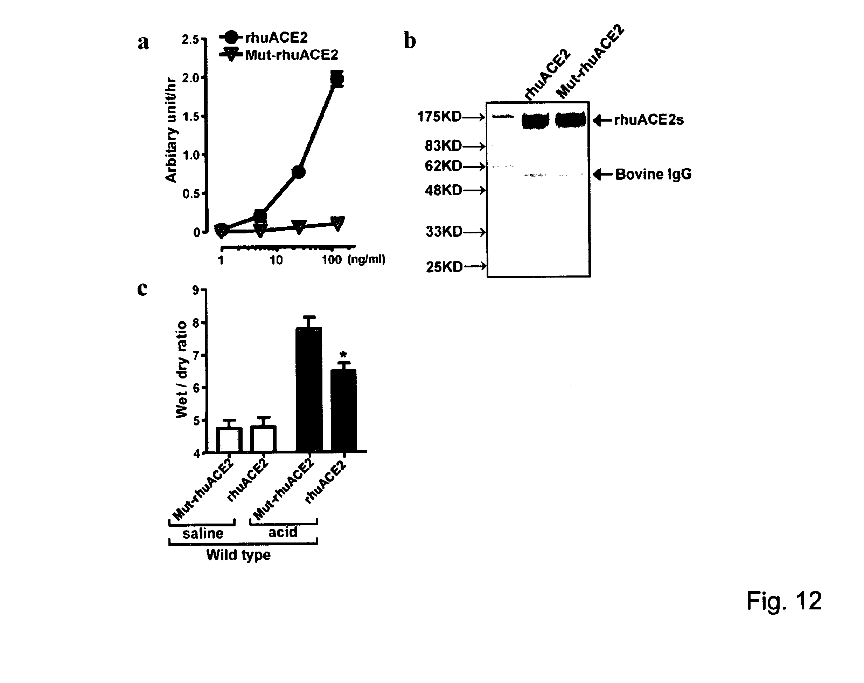

Figure 12. Recombinant ACE2 preparations and treatment of wild type mice with catalytically active and inactive ACE2: a, Catalytic activities of the extracellular domain (aa1-738) of recombinant human wild type ACE2 (rhuACE2) and mutated extracellular domain (aa1-738) of recombinant human ACE2 (Mut-rhuACE2) that carries two inactivating mutations in the catalytic domain (H374N & H378N). Activities of the purified proteins to cleave a specific fluorogenic substrate were measured in vitro (see Methods). b, Commassie blue-stained SDS-PAGE gel of rhuACE2 and Mut-rhuACE2 proteins purified from supernatants of transfected CHO cells. c, Wet to dry weight ratios of lungs in rhuACE2- and Mut-rhuACE2-treated WT mice in acid-induced ALI. n = 6-8 per group. Bars show mean values +/- s.e.m. Asterix, p < 0.05 (ANOVA and two-tailed t-test).

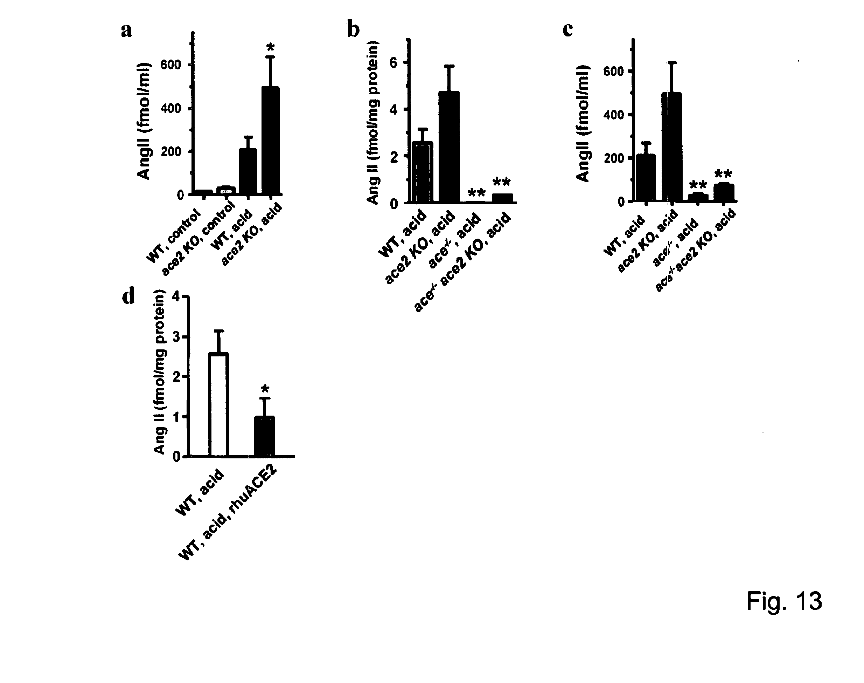

Figure 13. ACE2 negatively regulates AngII levels: a, Plasma AngII peptide levels in control and acid-treated WT and ace2 KO mice. AngII levels were determined at 3 hrs by EIA. Bars show mean values +/- s.e.m. (n = 3-5 per group). Asterisk, p < 0.05 (ANOVA and two-tailed t-test) between acid treated WT and ace2 KO mice. b, Plasma and c, lung Ang II peptide levels in WT, ace2 KO, ace^{-/-}, and ace^{-/-}ace2 double mutant mice in acid-induced ALI. AngII levels were determined at 3 hrs by EIA. Bars show mean values +/- s.e.m. (n = 3-5 per group). Double asterix, p < 0.01; (ANOVA and two-tailed t-test) comparing acid-treated ace2 KO with acid-treated ace^{-/-} single or ace^{-/-}ace2 double mutant mice. d, Lung AngII levels in acid challenged wild type mice treated with vehicle or rhuACE2. AngII levels were determined at 3 hrs by EIA. Bars show mean values +/- s.e.m. (n = 3-5 per group). Asterisk, p < 0.05 (ANOVA and two-tailed t-test).

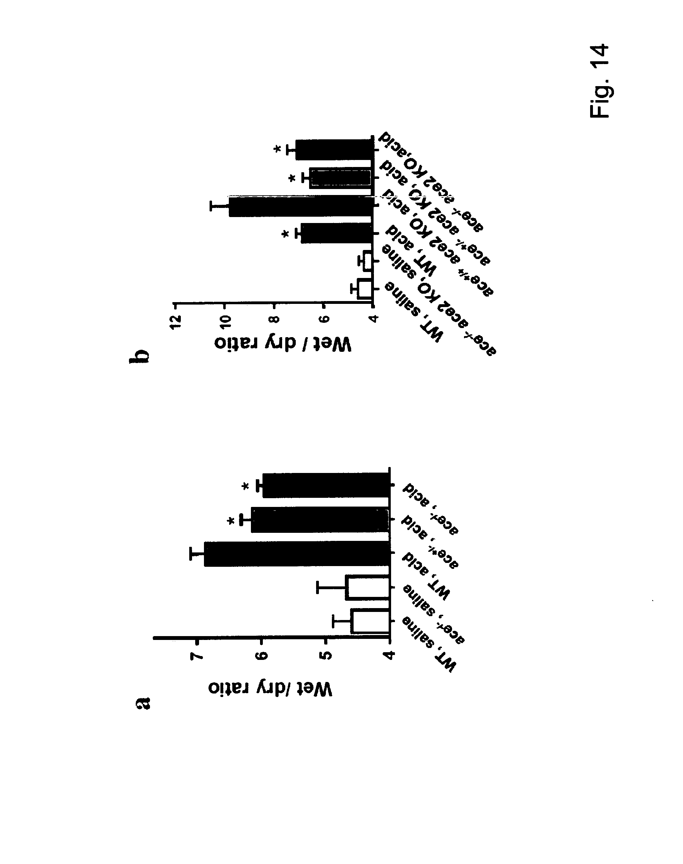

Figure 14. Genetic inactivation of ace attenuates lung oedema formation: a, Wet to dry weight ratio of lungs in WT, ace^{+/-}, and ace^{-/-} mice in acid-induced ALI and saline-treated ace^{+/+} WT and ace^{-/-} mice determined at 3 hrs. Bars show mean values +/- s.e.m. n = 4-6 mice per group. Asterisk, p < 0.05 (ANOVA and two-tailed t-test) comparing acid-treated WT with acid-treated ace^{+/-}, or ace^{-/-} mice. b, Wet to dry weight ratios of lungs in acid-treated ace2 KO, ace^{+/-}ace2 KO, ace^{-/-}ace2 KO and WT mice and saline-treated ace^{-/-}ace2 KO and WT mice determined at 3 hrs. Bars show mean values +/- s.e.m. n = 5 mice per group. Asterisk, p < 0.05 (ANOVA and two-tailed t-test) comparing acid-treated ace2 KO with acid-treated ace^{+/-}ace2 KO, ace^{-/-}ace2 KO or WT mice.

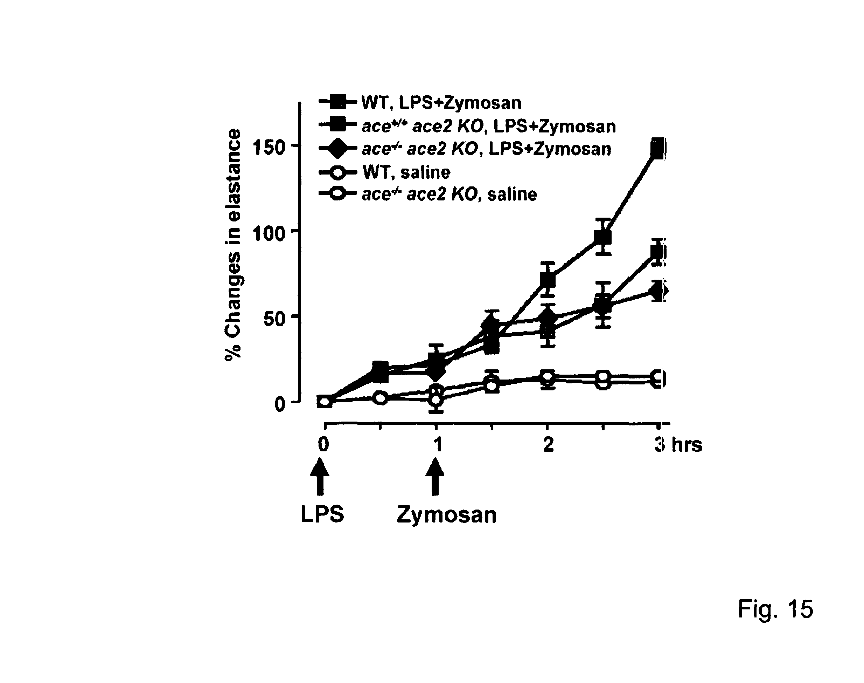

Figure 15. ace deficiency attenuates endotoxin-induced acute lung injury on an ace2 KO background: Lung elastance measurements in WT, ace2 KO, and ace^{-/-}ace2 KO mice after LPS plus zymosan challenge. The timing of LPS and zymosan instillations are indicated. n = 3-6 mice per group. p < 0.05 comparing ace2 KO with WT or ace^{-/-}ace2 double mutant mice at 3 hours (ANOVA and two-tailed t-test).

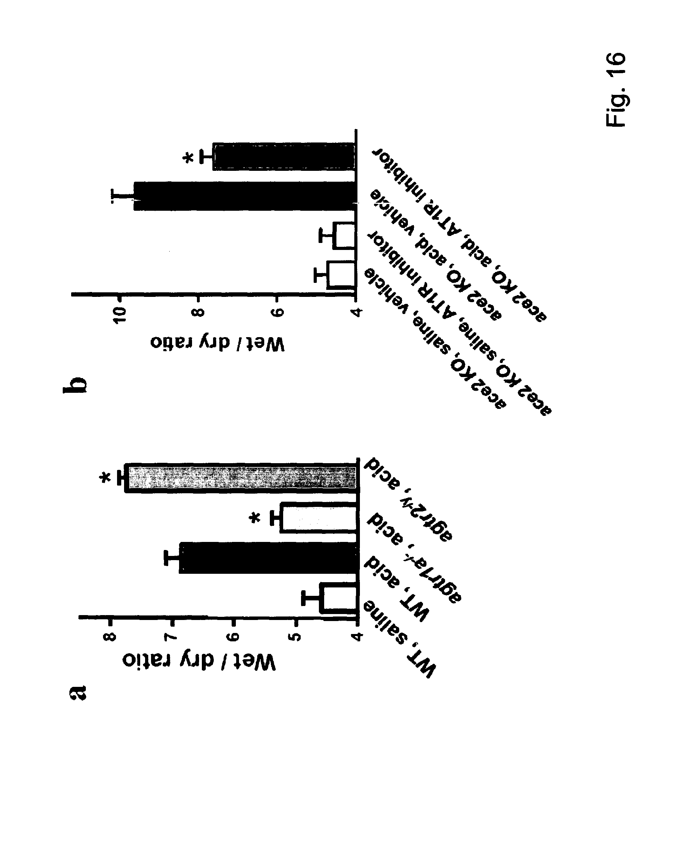

Figure 16. The AT1R mediates acid induced lung oedemas: a, Wet to dry weight ratios of lungs in agtr1a^{-/-}, agtr2^{-/y}, and WT mice. n = 4-6 mice per group. Bars show mean values +/- s.e.m. Asterisk, p < 0.05 comparing WT with agtrla^{-/-} and WT with agtr2^{-/y} mice (ANOVA and two-tailed t-test). Without injury, there were no differences in wet-to-dry weight ratio of lungs among WT, agtr1a^{-/-}, or agtr2^{-/y} mice. b, Wet to dry weight ratios of ace2 KO lungs in the presence or absence of the specific AT1R blocker Losartan (15 mg/kg). n = 4-6 mice per group. Bars show mean values +/- s.e.m. Asterisk, p < 0.05, comparing AT1R inhibitor-treated ace2 KO with vehicle-treated ace2 KO mice at 3 hrs after acid injury (ANOVA and two-tailed t-test).

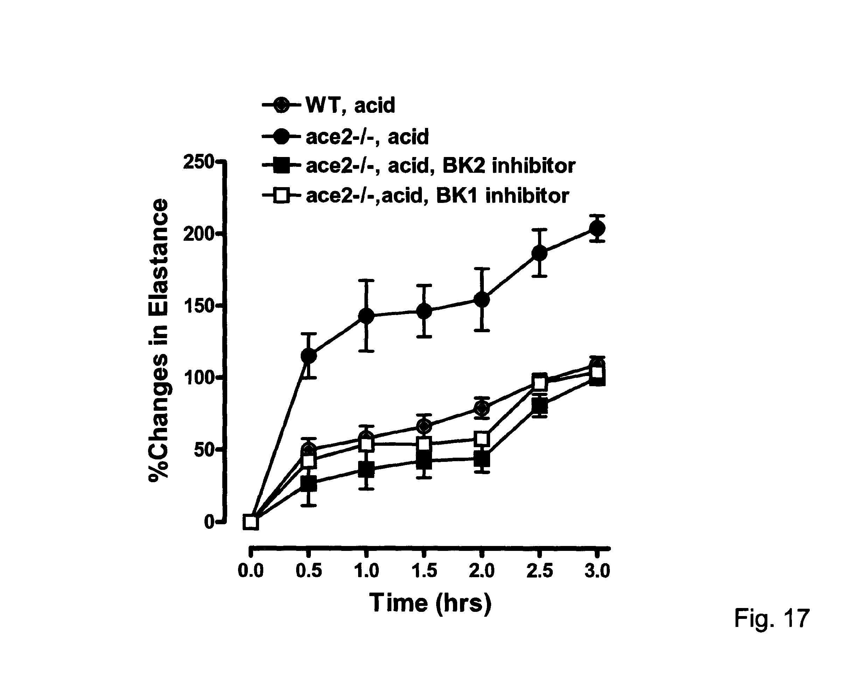

Figure 17. Effects of bradykinin receptor inhibitors in ALI measured by elastance: Addition of bradykinin receptor inhibitors worsens acid aspiration-induced acute lung injury: a, Lung elastance measurements after acid or saline treatment in wild type (WT) and ace2 knockout (ace2 KO) mice. n = 10 for each acid treated group. n = 6 for each saline control group. Asterisk, p < 0.05 for the whole time course comparing acid-treated WT and ace2 KO mice (repeated measurement ANOVA)

### Examples:

### Example 1.: ACE2 expression in mouse lungs

ACE2 expression has been primarily found in epithelial and endothelial cells of kidneys, heart, and human lungs. Therefore ACE2 expression in mouse lung tissue was analysed. Similar to humans, mouse ACE2 mRNA is expressed on vascular endothelial and airway epithelial cells in the lungs using in situ hybridizations (Fig. 1). In Western Blot analysis, a 125 kDa band for ACE2 protein was observed specifically in wild type mice but not in ace2 knockout animals (Fig 1b). Real time PCR showed that ACE expression was comparable between ACE2-expressing and ace2 knock-out mice. Loss of ACE2 did not affect basal lung functions (Fig. 1c) or lung structure (see Fig. 7). Similarly, ace single mutant, ace/ace2 double knock-out, agtr1a^{-/-}, and agtr2^{-/-} mice display normal lung structure and normal baseline lung functions.

### Example 2: A critical role of ACE2 in SARS pathogenesis

Here the first genetic proof is provided that ACE2 is a critical SARS receptor in vivo (Fig.2). Intriguingly, SARS infections and the Spike protein of the SARS coronavirus (see Fig.6) reduce ACE2 expression (Fig.3) and SARS-Spike injection into mice worsens ARDS symptoms in vivo (Fig.4). Importantly, Spike-mediated acute lung failure can be attenuated by modulating the renin-angiotensin pathway (Fig.5). These results provide a molecular explanation why SARS infections cause severe and often lethal lung failure and suggest a rational therapy for SARS and possibly other respiratory disease viruses, such as e.g. influenza, avian flu, anthrax, pneumococcal, ....

### Materials and methods

Mice: ace2 mutant mice were generated as described and back-crossed to C57Bl/6 more than 5 times. Only sex, age, and background matched mice were used as controls. Mice were genotyped by PCR and Southern blotting as described (Crackower et al., 2002) and maintained at the animal facilities of the Institute of Molecular Pathology, Vienna, and for SARS infections at the Institute of Laboratory Animal Sciences, Chinese Academy of Meidcal Sciences & Peking Union Medical College, Beijing, P.R. China, in accordance with each institutional guidelines. All SARS experiments were approved by the Chinese authorities.

Virus: the SARS-CoV (PUMC01 isolate, genbank access number AY350750) used in this study was kindly provided by Z. Wang and Y. Liu of PUMC hospital. This isolate was certified by National Institute for the Control of Pharmaceutical and Biological Products (No. SH200301298). The virus was isolated and passaged eight times to generate a virus stock with a titer of 10^{6.23} 50% tissue culture infective doses (TCID₅₀)/ml. All work with infectious virus was performed inside a biosafety cabinet, in a biosafety containment level 3 facility. All work with SARS-CoV PUMC01 isolate was proved by Ministry of Health and performed in the guidance of "Laboratory Biosafety Management of Pathogen" from state council of People's Republic of China.

Animal studies: The mouse studies were approved by the Ministry of Health Science and Technology division and were carried out in an approved animal biosafety level 3 facility. Female mice were housed less than four per cage and male mice were housed one per cage. Mice three to five weeks were lightly anesthetized with isoflurane were inoculated with 100 µl virus intranasally. On day 2 mice were euthanized with carbon dioxide, and the lungs were removed and frozen at -70°C. The frozen tissues were thawed and homogenized in a 10% suspension in DMEM medium (Invitrogen). Virus titers were determined in Vero cell monolayers in 24- and 96-well plates. Virus titers are expressed as TCID50 per gram of lung tissue. Total RNA and Protein isolated from part of homogenized lung tissues using Trizol reagent (Invitrogen) were frozen and stored at -70°C.

Real time RT-PCR: Standard protocols were used. The mouse beta-actin housekeeping gene was used for sample normalization.

SARS-Spike protein binding experiments: The coding sequence of SARS spike protein (amino acids 1-1190 from Urbani strain) or a Spike sequence that only contains the previously mapped (Li et al., 2003; Wang et al., 2004). ACE2 binding domain (aa318-510) were codon optimized, synthesized, and subcloned into the PEAK vector to generate a fusion protein with the Fc portion of human IgG1. CHO cells were transfected with the Spike-Fc expression vector, supernatants harvested, and Spike-Fc protein purified by affinity chromatography using a Protein A Sepharose column. For in vitro binding assays, A549 human alveolar epithelial cells or IMCD murine kidney epithelial cells were homogenized in lysis buffer (50 mM Tris-HCl, pH 7.4, 20 mM EDTA, and 1% Triton-X100) supplemented with "Complete" protease inhibitor cocktail (Roche) and 1 mM Na₃VO₄. Cell lysates were incubated with Spike-Fc or control human IgG-Fc protein with gentle agitation for 2 hours at 4°C. Spike-Fc or control human IgG-Fc protein were pulled down by Protein G Sepharose, proteins separated by SDS-polyacrylamide gel electrophoresis (PAGE), and transferred to a nitrocellulose membrane. Pulled-down human and mouse ACE2 were detected by anti-human ACE2 polyclonal antibodies (R&D systems) and an anti-mouse ACE2 polyclonal antibody (Crackower et al., 2000), respectively. For flow cytometry, Vero E6 cells are detached by 2mM EDTA/PBS and incubated with Spike-Fc or control human IgG-Fc protein at 4°C or 37°C for 3 hours. Cells were then incubated with anti-ACE2 monoclonal antibody, followed by FITC-conjugated anti-mouse IgG Abs (Jackson ImmunoResearch Laboratories, Inc). In addition, a FITC-conjugated anti-human IgG polyclonal antibody was used for detection of Spike-Fc protein and control IgG-Fc bound to Vero E6 cells. In both experimental systems, similar results were obtained. Full length mouse and human ACE2 coding regions were cloned into a PEAK vector and transfected into 293 cells. Spike-Fc binding was detected as above. All samples were analyzed by flow cytometry using a FACScan (Becton Dickinson).

Recombinant Spike-Fc challenge in mice with acid-induced acute lung injury: The mouse model of acid aspiration-induced acute lung injury was used (Imai et al., 2003) for Spike-Fc in vivo experiments. Mice (2.5-3 months old) received Spike (S1190)-Fc, Spike-(S318-510)-Fc (5,5 nmol/kg each) or control-Fc i.p. three time at 30 min before and at 1 and 2 hours after acid treatment. After HCl instillation, animals were randomized into the indicated experimental cohorts. All animals were then ventilated for 3 hrs and analyzed as described in Imai et al.. For AT1R inhibition of Spike-Fc-mediated acute lung injury, the Spike-Fc challenged mice were treated with the AT1R inhibitor Losartan (15 mg/kg). For histological analysis, 5-µm thick sections were cut and stained with hematoxylin and eosin (H&E). For detection of Angiotensin II peptide levels, lungs were homogenized on ice in 80% ethanol / 0.1% HCl containing peptidase inhibitors as described I detail by Imai et al..

Statistical analyses: All data are shown as mean ± s.e.m.. Measurements at single time points were analyzed by ANOVA and in case of significance further analyzed by a two-tailed t-test. Time courses were analyzed by repeated measurements (mixed model) ANOVA with Bonferroni post-t tests. All statistical tests were calculated using the GraphPad Prism 4.00 (GraphPad Software, San Diego, CA, USA) and a JMP (SAS Institute, Toronto, ONT, Canada) programmes. p < 0.05 was considered to indicate statistical significance.

### Results and Discussion

To address this question genetically, ace2 knock-out and control wild type mice were infected with the SARS-Coronavirus. As reported previously, SARS infections of wild type mice result in viral replication in the lungs and the recovery of large amounts (> 10⁷ TCID₅₀ per gram lung tissue) of infectious virus (Fig.2a,b). The SARS infection of mice is associated with the development of mild pathological changes in their lungs (Fig. 2c). In ace2 KO mice, only a very low number of infectious SARS virus could be recovered (< 10² TCID₅₀ per gram lung tissue) (Fig. 2a), and SARS replication was impaired using semiquantitative real time RT- PCR (Fig. 2b). Moreover, no obvious pathologic alterations in the lungs of ace2 mutant mice was detected (Fig. 2c). These data provide the first genetic proof that ACE2 is indeed a critical SARS receptor required for effective replication and recovery of infectious SARS virus.

Experimental SARS infections of wild type mice in vivo also resulted in significantly reduced ACE2 protein and mRNA expression in the lungs (Fig. 3a) and hearts showing that reduced ACE2 expression has a role in SARS-mediated severe acute lung pathologies in vivo. By contrast, ACE expression levels were not overtly changed in SARS infected mice (Fig. 3a). To test whether SARS might affect lung pathologies through ACE2 a defined model system using recombinant SARS coronavirus surface-Spike protein which is the essential ligand for ACE2 binding was established. Such a model system allows to avoid possible secondary effects due to viral replication/infections in vivo and to directly test whether SARS-Spike might adversely affect acute lung injury through modulation of ACE2.

It was first tested whether recombinant SARS Spike protein (Fig 6a) binds to human as well as murine ACE2 protein in using in vitro pull down assays. Recombinant Spike-Fc protein indeed pulled down both human and murine ACE2 (Fig. 3b). SARS Spike-Fc binding to human and mouse ACE2 was confirmed by FACS binding assays of Spike-Fc to 293 cells overexpressing human or murine ACE2 (Fig. 3c). Moreover, Spike-Fc binds to endogenous ACE2 in Vero E6 cells (Fig. 3d). Importantly, binding of Spike-Fc to endogenous ACE2 in Vero cells resulted in downregulation of ACE2 surface expression (Fig. 3d). Spike-Fc also decreased surface levels of human and mouse ACE2 overexpressed in 293 cells and triggered syncythia formation of murine ACE2 transfected but not control CD4 transfected 293 cells. Thus, analogous to other virus-receptor interactions, SARS Spike protein binding to ACE2 in cell lines or SARS infections in vivo results in reduced ACE2 protein expression.

Since ACE2 is a critical SARS receptor (Fig. 2), SARS-Spike protein binding to ACE2 downmodulates ACE2 expression (Fig. 3), it was tested whether SARS-Spike protein, which is the critical ACE2 binding protein, could affect the severity of acute lung injury in vivo. Intriguingly, treatment with Spike-Fc protein worsened the lung function in wild type mice, whereas control-Fc protein showed no apparent effects (Fig. 4a). Moreover, Spike-Fc treatment of acid-challenged wild type mice augmented the pathological changes in the lung parenchyma (Fig. 4b) and increased lung oedemas as defined by a wet/dry lung weight ratios (Fig. 4c). A Spike-deletion mutant was also made that only contains the previously mapped ACE2-binding domain (aa318-510) fused to human Fc (Fig. 4d). This short Spike(S318-510)-Fc protein also binds to ACE2 in cell lines using FACS assays and downmodulates ACE2 cell surface expression (Fig. 6b). Treatment of Spike(S318-510)-Fc again worsened acid-induced acute lung injury in wild type mice (Fig. 4d). Importantly, in vivo Spike-Fc protein administration did not affect the severity of lung failure in ace2 knockout mice (Fig. 5a), indicating that the effect of Spike protein on acute lung injury is ACE2 specific. These results show that the SARS coronavirus Spike protein can directly affect the development of severe acute lung failure via ACE2.

ACE2 functions as a carboxypeptidase, cleaving a single residue from AngI, generating Ang1-9, and a single residue from AngII to generate Ang1-7. The ACE2 homologue ACE, by contrast, cleaves the decapeptide AngI into the octapeptide AngII. Thus, ACE2 counter-balances the function of ACE and negatively regulates AngII production. To test whether Spike-Fc injections indeed affect the function of the renin-angiotensin system, AngII levels in the lungs of acid/Spike-Fc treated mice were analyzed. Acid aspiration increased AngII levels in the lungs of wild type mice. Importantly, a further, significant increase in AngII levels in the lung tissue of mice treated with Spike-Fc was observed (Fig. 5b). To further confirm whether Spike-Fc promotes lung disease pathogenesis through increased AngII production and functional alterations of the RAS, the AT1 receptor was blocked with a specific inhibitor. Inhibition of the AT1R indeed attenuated acute severe lung injury in Spike-Fc treated mice (Fig. 5c). Inhibition of the AT1R also attenuated pulmonary oedema of Spike-Fc treated mice (Fig. 5d). Taken together, the present data show that SARS Spike can exaggerate acute lung failure via deregulation of the angiotensin system. Moreover, SARS Spike mediates lung failure can be rescued by inhibition of the AngII receptor.
It has been estimated that lethality of the Spanish flu virus that killed more than 20 million people at the beginning of the 20th century was ~ 0.5 percent of infected people whereas the lethality of SARS coronavirus infections reached 10% even with modern intensive care treatment. Due to this very high lethality of SARS and the enormous economic and social impact of the worldwide SARS outbreak, elucidation of the disease pathogenesis is critical for future treatment in case of renewed outbreaks. Moreover, a recent outbreak of avian influenza A (H5N1) in humans also resulted in up to 70% of lethality due to acute respiratory failure. Before the discovery of the SARS coronavirus, two coronaviruses (HCoV-229E and HCoV-OC43) were known to infect humans, but they caused only self-limiting upper respiratory tract infections (30% of the common colds) and had never been reported to cause severe illness. The molecular determinants that may account for the dramatic differences in pathogenesis between the human coronaviruses (HCoV-229E, HCoV-OC43) and SARS-coronavirus were unknown.

The data according to the present invention provide a molecular explanation for the severe lung failure and probably lethality associated with SARS: infections with the SARS coronavirus result in ACE2 downregulation through binding of SARS Spike protein to ACE2. Since ACE2 is a critical negative regulatory factor for severity of lung oedema and acute lung failure, SARS Spike protein mediated ACE2 downregulation then contributes to the severity of lung pathologies. This scenario would explain how this novel family member of the "relatively harmless" coronaviruses has turned into a lethal virus.

The data according to the present invention provide a molecular link between SARS pathogenesis and the role of RAS in lung failure. Recombinant ACE2 protein therefore is not only a treatment to block spreading of SARS but modulation of the renin-angiotenins system can also be utilized to protect SARS patients, and possibly patients infected with other viruses and other infectious diseases such as avian influenza A strains, from developing acute severe lung failure and ARDS.

### Example 3: The SARS-coronavirus receptor ACE2 protects from severe acute lung failure, lung blood vessel permeability and lung oedemas

The results in this example show that ACE2 protects mice from severe acute lung injury induced by acid aspiration or sepsis. Disease pathogenesis was mapped to the ACE-angiotensin II-angiotensin II type-1a receptor (AT1aR) pathway while ACE2 and the angiotensin II type-2 receptor mitigate acute lung failure. Mechanistically, the ACE-AngII-AT1aR axis induces increased lung oedemas and impaired lung function. These data identify a critical function for ACE2 in acute lung injury, lung blood vessel permeability and lung oedemas. Furthermore, it was shown that recombinant human ACE2 protects mice from severe acute lung injury, lung blood vessel permeability and lung oedemas, suggesting a novel therapy for an often lethal and previously untreatable syndrome that affects millions of people worldwide/year with different diseases such as sepsis or pneumonias including SARS and influenza patients.

### Methods:

Mice and materials: ace2, ace, agtrla, and agtr2 mutant mice have been previously generated and were genotyped as described (Crackower et al., 2002; Sugaya et al., 1995; Hein et al., 1995; Krege et al., 1995). ace mutant mice were obtained from the Jackson Laboratories. Double mutant mice were generated by intercrosses. Only sex, age, and background matched mice were used as controls. Basal lung functions and lung structure were comparable among all the mice tested. Mice were genotypes by PCR and Southern blotting and maintained in accordance with institutional guidelines.

Experimental murine ALI models: For acid aspiration-induced ALI, 2.5-3 month old mice were anaesthetized with ketamin (75 mg/kg) and xylazine (20 mg/kg) i.p., tracheostomized and ventilated with a volume control constant flow ventilator (Voltek Enterprises, Canada). Volume recruitment manoeuvre (VRM) (25 cmH₂O, 3 sec) was performed to standardize volume history and measurements were made as baseline. After intratracheal instillation of HCl (pH=1.5; 2 ml/kg), followed by a VRM (35 cmH₂O, 3 seconds), animals were ventilated for 3 hrs (F_{I}O₂ 1.0). Saline-treated groups served as controls. For endotoxin-induced ALI, anesthetized and mechanically ventilated mice received 0.5 µg/g of LPS from E. coli 0111:B4 (Sigma Chemical Co., St.Louis, MO) and 3 µg/g of zymosan A from Saccharomyces cerevisiae (Sigma) intratracheally immediately after starting mechanical ventilation and one hour later, respectively. Saline-treated groups served as controls. To study sepsis induced ALI, cecal ligation perforation (CLP) was performed as previously described (Martin et al., 2003). Briefly, a midline incision was performed in the abdomen of anesthetized mice. The cecum was isolated and ligated 5.0 mm from the cecal tip. The cecum was then punctured twice with an 18-gauge needle, and stool was extruded (1mm). After repositioning of the cecum, the abdomen was closed with a 4-0 silk suture. Sham-operated mice underwent the same procedure without ligation and puncture of the cecum. All animals were monitored throughout an 18-hrs recovery period. Thereafter, animals were subjected to mechanical ventilation for up to 6 hrs as described above. In all experimental ALI models, total PEEP (PEEPt) and plateau pressure (Pplat) were measured at the end of expiratory and inspiratory occlusion, respectively, and elastance was calculated as Pplat minus PEEPt)/V_{T} every 30 minutes during the ventilation periods. At the end of the ventilation, left lungs were sampled for the measurement of lung wet/dry mass ratios or snap frozen in liquid nitrogen for subsequent biochemical analysis, and right lungs were fixed in 10 % buffered formalin for histological examination.

Assessment of blood oxygenation, pulmonary oedema, and pulmonary vascular permeability: At the end of the experiments, blood samples were obtained from the left heart ventricle and PaO2 was measured (Ciba-Corning Model 248, Bayer, Leverkusen, Germany) to assess arterial blood oxygenation as an indicator for respiratory failure. To assess pulmonary oedemas, the lung wet/dry weight ratios were calculated. In brief, after the blood was drained from the excised lungs, measurements of the lung wet weight were made. Lungs were then heated to 65°C in a gravity convection oven for 24 hrs and weighed to determine baseline lung dry mass levels. Pulmonary vascular permeability was assessed by measuring the pulmonary extravasation of Evans Blue (Goggel et al., 2004) or using Dextran-FITC. Evans Blue (20 µg/g) was injected into the jugular vein at the end of the 3 hrs ventilation period. Ten minutes after the injection of Evans Blue, the animals were sacrificed. Lungs were then perfused with ice-cold PBS before the lung tissue was used to determine the content of Evans Blue.

Histology and in situ hybridizations: For histological analysis, 5-µm thick sections were cut and stained with haematoxylin and eosin (H&E). For in situ hybridization, lungs were fixed for 24 hours in 4% buffered formalin and sectioned at 5µm. Strand-specific sense and antisense riboprobes for ACE2 were generated by incorporating digoxygenenin (DIG)-labeled UTP (Boehringer Manheim, Laval, PQ, Canada). DIG in situ hybridization was performed essentially as described (Griffiths et al., 2003).

Recombinant ACE2 treatments in ALI: The mouse acid aspiration-induced ALI model (see above) was used for ACE2 rescue in vivo experiments. Thirty minutes before acid instillation, mice (2.5-3 months old) received either recombinant human ACE2 (rhuACE2) protein (0.1 mg/kg), catalytically inactive mutant recombinant human ACE2 (Mut-rhuACE2), or vehicle (0.1% BSA/PBS) i.p.. All animals were then ventilated for 3 hrs and analyzed as described above. To prepare recombinant human ACE2 protein (rhuACE2), the coding sequence of the extracellular domain (aal-738) of human ACE2 was subcloned into the PEAK vector to generate a fusion protein with the Fc portion of human IgG1 (rhuACE2). For Mut-rhuACE2, two inactivating mutations in the catalytic domain (H374N & H378N) (Li et al., 2003) were introduced to the extracellular domain (aa1-738) of recombinant human ACE2 using site directed mutagenesis. CHO cells were transfected with the rhuACE2 and Mut-rhuACE2 expression vector, supernatants harvested, and rhuACE2 protein and Mut-rhuACE2 protein purified by affinity chromatography using a Protein A Sepharose column. Commassie-stained gels showed that the purity of rhuACE2 or Mut-rhuACE2 was approximately 90% due to the co-purification of bovine IgG from culture media (Figure 12b). The catalytic activities of purified recombinant ACE2 proteins were measured essentially as described previously (Huang et al., 2003) by using the fluorogenic peptide Substrate VI (FPS VI, 7Mca-Y-V-A-D-A-P-K (Dnp)-OH, R&D Systems, Minneapolis, MN). Briefly, the reactions were done in a buffer containing 0.1%BSA, 1 M NaCl, 75 mM Tris, 0.5 mM ZnCl₂, pH 7.5 in a final reaction volume of 100 µl at 37° C. The change in fluorescence was monitored using a Fluoroskan II Fluorescence Reader (Global Medical Instrumentation, Inc, MN). Mut-rhuACE2-Fc showed more than 95% loss of catalytic activity (Figure 12a). Lung functions and lung oedema formation were improved in ace2 KO and wild type mice using recombinant ACE2 protein.

AT1R/AT2R inhibitor studies: Mice (2.5-3 months old) received the AT1R inhibitor Losartan (15 mg/kg), the AT2R inhibitor PD123.319 (15 mg/kg), or control vehicle i.p. 30 min before surgical procedures. After HCl instillation, animals were randomized into 4 groups: (1) ace2 KO, acid, vehicle control; (2) ace2 KO, acid, AT1R inhibitor; (3) ace2 KO, acid, AT2R inhibitor; (4) WT, saline, vehicle control. Animals were then ventilated for 3 hrs and analyzed as above.

Detection of Angiotensin II peptide levels: Lungs were homogenized on ice in 80% ethanol / 0.1% HCl containing peptidase inhibitors as described (Crackower et al., 2002). Protein homogenates were centrifuged at 30,000g for 20 minutes, supernatants decanted, and acidified with 1% (v/v) heptafluorobutyric acid (HFBA, Pierce, Rockford, IL). The supernatant was concentrated to 5 ml on a Savant vacuum centrifuge (Savant, Farmingdale, NY) and concentrated extracts were applied to activated Sep-Paks, washed with 0.1% HFBA, and eluted with 5 ml 80% methanol / 0.1% HFBA. Analysis of angiotensin peptide content in the lung extracts and plasma were performed using Enzyme ImmunoAssay (Spi-Bio).

Ex vivo perfused mouse lungs: Mouse lungs were prepared as described (Goggel et al., 2004). Briefly, the isolated mouse lungs were ventilated (V_{T} of ~8 ml/kg, 90 breaths•min-1) and perfused in a non-recirculating fashion with RPMI medium containing 4% albumin at a constant flow of 1 ml•min-1. AngI or AngII were given as bolus injections (15 µg/kg) into the pulmonary artery and pulmonary artery pressure was measured to compare the hydrostatic responses. In one set of experiments animals were pretreated with acid instillation 60 min prior to the first injection of AngI or Ang II. In another set of experiments performed in untreated animals the first challenge was followed by repeated angiotensin injections in the presence of continuous LPS (10µg/ml) perfusion.

Echocardiography and invasive haemodynamics: Echocardiographic assessments were performed as described (Crackower et al., 2002) using wild-type and mutant littermates. Anesthetized mice described above were examined by transthoracic echocardiography using a Sonos 5500 (Philips Ultrasound) equipped with an 8 to 12MHz linear transducer. Fractional shortening (FS) was calculated as: FS = [(EDD - ESD)/EDD]*100. For arterial blood pressure measurements, the right carotid artery was cannulated with 1.4 French catheter and arterial blood pressure was monitored using a pressure transducer (Harvard Instruments).

Statistical analyses: All data are shown as mean ± s.e.m.. Measurements at single time points were analyzed by ANOVA and in case of significance further analyzed by a two-tailed t-test. Time courses were analyzed by repeated measurements (mixed model) ANOVA with Bonferroni post-t-tests. All statistical tests were calculated using the GraphPad Prism 4.00 (GraphPad Software, San Diego, CA, USA) and a JMP (SAS Institute, Toronto, ONT, Canada) programs. p < 0.05 was considered to indicate statistical significance.

### Results and Discussion:

The renin-angiotensin system (RAS) plays a key role in maintaining blood pressure homeostasis, as well as fluid and salt balance. Angiotensin converting enzyme 2 (ACE2) is a homologue of ACE, and functions as negative regulatory component of RAS. ACE2 has also been identified as a receptor for the Severe Acute Respiratory Syndrome (SARS)-coronavirus in cell lines. The mortality following SARS-coronavirus infections approached almost 10% due to the development of the ARDS. Although ACE2 is expressed in lungs of humans and mice (see Example 1 above), nothing was known about the function of ACE2 in lungs. To elucidate the role of ACE2 in acute lung injury (ALI) the impact of ace2 gene deficiency was examined in experimental models that recapitulate the common lung failure pathology observed in multiple human diseases including sepsis, acid aspiration, or pneumonias such as SARS and avian influenza A.

Aspiration of gastric contents containing a low pH is a frequent cause of ALI/ARDS. Experimental acid aspiration displays many characteristics of human ALI, i.e., hypoxemia, pulmonary oedema, and stiff lungs. Acid aspiration in wild type mice resulted in a rapid impairment of lung functions with increased lung elastance (Fig. 7a), decreased blood oxygenation (hypoxemia) (Fig. 7b), and the development of pulmonary oedema as defined by wet/dry lung weight ratios (Fig. 7c). Histologically, acid aspiration resulted in alveolar wall thickness, oedema, bleeding, inflammatory cell infiltrates, and formation of hyaline membranes (Fig. 7d). Intriguingly, acid-treated ace2 KO mice demonstrated a significantly greater lung elastance compared to control wild type mice, while there were no differences in lung elastance between saline-treated ace2 KO and WT mice (Fig. 7a). Moreover, loss of ace2 resulted in worsened oxygenation (Fig. 7b), massive lung oedema (Fig. 7c), and marked histological changes including increased inflammatory cell infiltration, pulmonary oedema, or hyaline membrane formations (Fig. 7d). Importantly, ACE2 protein expression is downregulated in wild type mice following acid challenge (Fig. 7e).

Sepsis is the most common cause of ALI/ARDS. To extend the present results, the impact of ace2 gene deficiency on sepsis-induced ALI was therefore examined using cecal ligation and perforation (CLP) model (Martin et al., 2003). CLP causes lethal peritonitis and sepsis due to a polymicrobial infection, which is accompanied by acute lung failure. In the CLP model, animals were subjected to mechanical ventilation 18 hours after the initial injury or sham operation as described in Martin et al., 2003. Whereas all (n=10) CLP-treated wild type mice survived, only 2 out of 10 CLP-treated ace2 mutant mice survived during the 6 hrs of experimental observation (Fig. 8a). Similar to acid aspiration, CLP resulted in lung failure defined by increased elastance (Fig. 8a), pulmonary oedema (Fig. 8b), and leukocyte accumulation (Fig. 8c) in wild type control mice. CLP-treated ace2 mutant mice again demonstrated a marked worsening of lung functions (Fig. 8a), increased oedema formation (Fig. 8b), and leukocyte accumulation (Fig. 8c), while there were no differences in these parameters between sham-treated ace2 KO and sham-treated WT mice (Fig. 8a-c). In addition, ALI was triggered with combined administration of lipopolysaccharide (LPS) and zymosan, which also mimicks human sepsis-associated ALI characterized by infiltration of inflammatory cells, pulmonary oedema, and deterioration of gas exchange. ace2 mutant mice again developed markedly enhanced acute lung injury following endotoxin challenge (Fig. 11a-c). Since ace2 maps to the X-chromosome, it should be noted that loss of ACE2 expression resulted in equally severe ALI phenotypes in males and females. These data in three different ALI models show that loss of ace2 expression precipitates severe acute lung failure.

To test whether loss of ACE2 is indeed essential for disease pathogenesis or alternatively results in a developmental compensation that then regulates ALI, an acute rescue experiment was performed using recombinant human ACE2 protein (rhuACE2) (Fig. 12a,b). Injection of rhuACE2 into acid-treated ace2 mutant mice indeed decreased the degree of acute lung injury assessed by lung elastance (Fig. 8d) and pulmonary oedema formation (Fig. 8e). When rhuACE2 protein was injected into acid treated wild type mice, it also improved lung function (Fig. 8f) and oedema formation (Fig. 12c). In saline-treated control wild type or ace2 mutant mice, injections of rhuACE2 did not affect pulmonary functions (Fig. 8d-f). Importantly, catalytically inactive ACE2 protein (Mut-rhuACE2) (Fig. 12a,b) did not rescue the severe lung phenotype in ace2 KO mice (Fig. 8d,e) and had no effect on the severity of ALI in wild type mice (Fig. 8f and Fig. 12c). These results show that ACE2 can directly protect lungs from acute lung injury through its catalytic activity.

ACE2 is a homologue of ACE and both are central enzymes in the RAS. Whereas ACE cleaves the decapeptide AngI into the octapeptide AngII, ACE2 functions as a carboxypeptidase, cleaving a single residue from AngI, generating Ang1-9, and a single residue from AngII to generate Angl-7. Thus, ACE2 regulates the RAS through inactivation of AngII and that the balance between ACE and ACE2 expression determines AngII production (Fig. 9a). Acid aspiration in wild type mice resulted in marked downregulation of ACE2 protein whereas ACE levels remain constant (Fig. 7e). Moreover, only catalytically active ACE2 improved the acute lung injury phenotype in mutant and wild type mice (Fig. 8d-f). To clarify whether acute lung injury indeed shifts the functional equilibrium between ACE and ACE2, AngII levels were measured in lungs and plasma of acid-treated and control mice. Acid aspiration markedly increased AngII levels in lungs (Fig. 9b) and plasma (Fig. 13a) of wild type mice. Importantly, a further, significant increase in AngII levels was observed in lungs (Fig. 9b) and plasma (Fig. 13a) of acid-treated ace2 KO mice. Thus, acute severe lung injury results in decreased ACE2 expression and increased production of AngII.

Therefore, in contrast to ACE2, ACE promotes disease pathogenesis through increased AngII production (Fig. 9a). Genetic inactivation of ace on an ace2 wild type and ace2 mutant background indeed markedly decreased AngII lung and plasma levels in the acid injury model (Fig. 13b,c). Moreover, treatment with rhuACE2 protein which attenuated lung injury (Fig. 8d-f) also reduced AngII levels in the lungs of acid treated mice (Fig. 13d). These data confirm that ACE is the enzyme responsible for increased AngII production and that ACE2 counterbalances the functions of ACE. Therefore lung injury in ace knockout mice was assessed. In contrast to ace2 mutants, ace^{-/-} mice were partly protected against acid-aspiration induced lung injury (Fig. 9c and Fig. 14a). Mice homozygous for the ace mutation displayed improved lung functions following acid aspiration compared to wild type controls (Fig. 9c). Importantly, these effects were gene dosage dependent and already observed in ace^{+/-} heterozygous mice that displayed a phenotype similar to ace^{-/-} mice. In addition, inactivation of ace on an ace2 knockout background rescued the severe lung failure phenotype (Fig. 9d), oedema formation (Fig. 14b), and histological changes (Fig. 9e) as compared to ace2 single mutants. Again, inactivation of only one ace allele (ace^{+/-}) could rescue the severe lung failure of ace2 KO mice (Fig. 9d,e). Similarly, in LPS/Zymosan-induced ALI, the severe lung impairments of ace2 KO mice were reversed by additional ace gene deficiency (Fig. 15). These data show that ACE promotes and ACE2 alleviates ALI pathology.

Both ACE and ACE2 are non-specific proteases that cleave additional substrates. Thus, although increased levels of AngII have been correlated with ace2 deficiency, it has never been shown that upregulation of the AngII pathway indeed accounts for the observed in vivo phenotypes of ace2 mutant mice. The receptors for AngII in mice are angiotensin II receptor type 1a (AT1aR), type 1b (AT1bR), and type 2 (AT2R). The lungs express ATlaR and AT2R, but not AT1bR. It was therefore explored which AngII receptor subtypes are responsible for ACE/ACE2 regulated ALI and whether AngII signalling through its receptors is responsible for ACE2 regulated lung pathology (Fig. 9a). To address these questions, mice that carry mutations in the ATlaR were challenged (agtr1a^{-/-}) and AT2R (agtr2^{-/Y}) with acid aspiration. Compared to wild type mice, genetic loss of AT1aR expression markedly attenuated ALI as determined by improved lung functions (Fig. 10a) and reduced oedema formation (Fig. 16a). Genetic inactivation of the AT2R markedly aggravated acute lung failure (Fig. 10a and Fig. 16a) resembling the severe ALI observed in ace2 mutant mice. Of note, acid aspiration-induced AngII levels in both agtr1a^{-/-} and agtr2^{-/Y} mice were comparable to that of wild type controls indicating that the observed effects were not secondary to altered AngII production but due to loss of receptor expression. Next it was attempted to rescue the severe lung injury phenotype of ace2 mutants using specific AT1R and AT2R blockers. Inhibition of the AT1R attenuated the severity of ALI in ace2 KO mice (Fig. 10b). By contrast, pharmacological inhibition of the AT2R had no apparent effect on the severe acute lung injury phenotypes of ace2 mutants (Fig. 10b). Inhibition of the AT1R in ace2 KO mice also attenuated acid-induced pulmonary oedema (Fig. 16b). Taken together, these data according to the present invention show that AT1aR and AT2R have opposite functions in controlling ALI severity and that angiotensin II through its AT1aR plays a causative role in acute lung failure.

Pulmonary oedema could either arise from augmented hydrostatic pressure due to pulmonary vascular constriction and/or enhanced microvascular permeability. It was first tested whether AngII can increase hydrostatic pressure using a murine isolated blood-free perfused ex vivo lung system to analyse pulmonary perfusion pressures under defined conditions. In this system, pulmonary perfusion pressures were comparable between wild type and ace2 KO mice under baseline control conditions (control wild types 3.0±1.9 cm H₂O, n=6 vs ace2 KO 1.8±1.6 cm H₂O, n=9; mean ± s.d.), and these values were not changed by either acid-treatment or during continuous LPS perfusion. Furthermore, pulmonary perfusion pressures generated by AngI or AngII injection in lungs from acid-instilled animals or in lungs perfused with LPS were also similar among wild type and ace2 KO mice (Fig. 17a,b). Moreover, fractional shortening using echocardiography (as an indicator for left ventricular systolic function) and mean arterial pressures using invasive haemodynamic measurements were comparable between ace2 KO and wild type mice during the experimental period. Thus, the severe lung oedemas in ace2 KO mice do not appear to be secondary to systemic haemodynamic alterations.

Since enhanced pulmonary vascular permeability is a hallmark of ALI/ARDS in human patients, it was next examined whether loss of ace2 results in increased vascular permeability in ALI using Evans Blue injections as an in vivo indicator for albumin leakage from the vasculature. Saline-treated control wild type and ace2 KO mice showed similar and very low vascular permeability as determined by accumulation of Evans Blue (Fig. 10c). Acid aspiration increased vascular permeability in wild type mice. Importantly, in ace2 mutant animals, pulmonary Evans Blue accumulation was greatly augmented following acid aspiration (Fig. 10c,d). These results were confirmed using FITC-conjugated Dextran (40kDa). To provide direct evidence that the increased permeability is caused by deregulated AngII signalling, vascular permeability in agtrla KO mice was measured. Whereas acid aspiration resulted in enhanced vascular permeability in wild type mice, vascular permeability was significantly attenuated in lungs of agtrla KO mice (Fig. 10e). Thus, in acute lung injury loss of ACE2 expression leads to leaky pulmonary blood vessels via AngII induced ATlaR stimulation.

Acute respiratory distress syndrome (ARDS) is the most severe form.of a wide spectrum of pathological processes designated as acute lung injury (ALI). ARDS is characterized by pulmonary oedema due to increased vascular permeability, accumulation of inflammatory cells, and severe hypoxia. Predisposing factors for ARDS are diverse and include sepsis, aspiration, pneumonias including infections with SARS coronavirus or avian and human influenza viruses. The data according to the present invention show that acute lung injury results in a marked downregulation of ACE2, a key enzyme involved in the regulation of the RAS. Injury triggered deregulation of the RAS then shifts the balance towards increased Angiotensin II levels. Intriguingly, functional differences can be already observed in mice heterozygous for the ace mutation. However, other ACE2 peptide metabolites such as bradykinin might play important roles in vivo, since inhibition of both bradykinin 1 (BK1) and bradykinin 2 (BK2) receptors also attenuate lung injury and lung failure (Fig.17), the present data also provide the first genetic confirmation that ACE2 regulated functions are indeed mediated through AngII.

It has been previously shown that an insertion/deletion (I/D) ACE polymorphism that affects ACE activity is associated with ARDS susceptibility and outcome. The present data provide a mechanistic explanation for these clinical findings and indicate that, in the pathogenesis of ALI, AngII is upregulated by ACE and drives severe lung failure via the ATlaR receptor. On the other hand, ACE2 and the AT2R regulate opposing effects and have protective roles against lung injury. Importantly, exogenous recombinant human ACE2 as well as inhibition of the AT₁-R or ACE attenuates acute lung failure in ace2 KO as well as wild type mice. These genetic, pharmacological, and ACE2 protein rescue experiments define a novel and critical role for the renin-angiotensin systems in the pathogenesis of acute lung injury and lung oedemas and demonstrate that ACE2 and its metabolites AngII and bradykinin (as well as bradykinin cleavage derivates) are key molecules involved in the development and progression of acute lung failure, lung vascular permeability and lung oedemas.

### Example 4: Effect of bradykinin receptors on acute lung injury

In this example the effect of bradykinin receptors (BK1, BK2) inhibitors on acute lung injury (ALI) was examined using murine acid-aspiration induced-ALI model. In particular, it was tested whether bradykinin receptors inhibitors rescued the phenotype on ace2 knock out (KO) mice.

### Methods:

Murine acid aspiration-induced lung injury model: For acid aspiration-induced ALI, 2.5-3 month old mice were anaesthetized with ketamin (75 mg/kg) and xylazine (20 mg/kg) i.p., tracheostomized and ventilated with a volume control constant flow ventilator (Voltek Enterprises). Volume recruitment manoeuvre (VRM) (25 cmH₂O, 3 sec) was performed to standardize volume history and measurements were made as baseline. After intratracheal instillation of HCl (pH=1.5; 2 ml/kg), followed by a VRM (35 cm-H2O, 3 seconds), animals were ventilated for 3 hrs (F_{I}O₂ 1.0). Total PEEP (PEEPt) and plateau pressure (Pplat) were measured at the end of expiratory and inspiratory occlusion, respectively, and elastance was calculated as Pplat minus PEEPt)/V_{T} every 30 minutes during the ventilation periods.

Bradykinin receptors inhibitor studies: Ace2 KO mice (2.5-3 months old) received the BK1 inhibitor (des Arg HOE, Sigma, 1.5 mg/kg), the BK2 inhibitor (HOE 140, Sigma, 1.5 mg/kg), or control vehicle i.p. 30 min before surgical procedures. After HCl instillation, animals were randomized into groups: (1) ace2 KO, acid, vehicle control; (2) ace2 KO, acid, BK1 inhibitor; (3) ace2 KO, acid, BK2 inhibitor. Animals were then ventilated for 3 hrs and analyzed as above.

### Results

Both BK1 inhibitor and BK2 inhibitor improved the lung failure assessed by pulmonary elastance in ace2 KO mice (Figure 17).

### References

Li, W. et al., Nature 426, 450-4 (2003)
Wang, P. et al., Biochem Biophys Res Commun 315, 439-44 (2004) Crackower et al., Nature 417, 822-8 (2002)
Imai, Y. et al., Jama 289, 2104-12 (2003)
Zou, K et al., ACTA Academiae Medicinae Sinicae. 25, 495-498 (2003)
Itoyama et al., Biochem Biophys Res Commun 323, 1124-9 (2004) Martin et al., Am J Physiol Lung Cell Mol Physiol 285, L1222-32 (2003)
Sugaya et al., J Biol Chem 270, 18719-22 (1995)
Hein et al., Nature 377, 744-7 (1995)
Krege et al., Nature 375, 146-8 (1995)
Martin et al., Am J Physiol Lung Cell Mol Physiol 285, L1222-32 (2003)
Goggel et al., Nat Med 10, 155-60 (2004)
Griffiths et al., Mol Cell Biol 23, 7708-18 (2003)
Huang et al., J Biol Chem. 278, 15532-40 (2003)
Coulie, Mol. Med. Today 3, 261-268 (1997)
Klink et al., J Cyst Fibros. 3 Suppl 2:203-12 (2004)
Alexander et al., Brit.J.Pharmacol.141, Suppl.1 (2004)
Burgess et al., Brit,J.Pharmacol.129, 77 - 86 (2000)
Heitsch, Curr Med Chem. 2002 May;9(9):913-28
Grenlee, Pharm. Res., 4, 364-374 (1987)

## Claims

1. : Use of Angiotensin converting enzyme 2 (ACE2) for the preparation of a medicament for the treatment of severe acute lung injury, especially induced by acid aspiration or sepsis, of lung oedemas and lung injuries and failures connected with infection with severe acute respiratory syndrome (SARS) coronavirus.

2. : Use of a combination of ACE2 and an AT₁-inhibitor for the preparation of a medicament for the treatment of lung injuries or lung diseases.

3. : Use according to claim 1 or 2 **characterised in that** the medicament comprises recombinant ACE2.

4. : Use according to any one of claims 1 to 3, **characterised in that** the medicament comprises an AT₁-inhibitor selected from the group consisting of candesartan, eprosartan, irbesartan, losartan, telmisartan, valsartan, olmesartan, tasosartan, embusartan, forsartan, milfasartan, pratosartan, ripisartan, saprisartan, zolasartan or mixtures thereof, especially telmisartan, or pharmaceutically acceptable salts thereof.

5. : Use according to any one of claims 1 to 4, **characterised in that** the medicament comprises a nucleic acid encoding ACE2 in addition or instead of ACE2.

6. : Use according to any one of claims 1 to 5, **characterised in that** the medicament comprises an ACE inhibitor.

7. : Use according to any one of claims 1 to 6, **characterised in that** the medicament comprises an ACE inhibitor selected from the group consisting of benazepril, captopril, ceronapril, enalapril, fosinopril, imidapril, lisinopril, moexipril, quinapril, ramipril, trandolapril, perindopril, alacepril, cilazapril, delapril, spirapril, temocapril, zofenopril or mixtures thereof, or pharmaceutically acceptable salts thereof.

8. : Use according to any one of claims 1 to 7, **characterised in that** the medicament comprises a bradykinin receptor inhibitor, especially a des-Arg⁹-bradykinin-inhibitor.

9. : Use according to any one of claims 1 to 8, **characterised in that** the medicament comprises a bradykinin receptor inhibitor selected from the group consisting of Lys-Lys [Hyp³,Cpg⁵,dTic⁷,Cpg⁸]des-Arg⁹]-bradykinin (B9958), AcLys-Lys([αMe] Phe⁵,D-βNal⁷,Ile⁸]des-Arg⁹-bradykinin (R914), AcLys[D Nal⁷,Ile⁸] [des-Arg⁹]-bradykinin(R715), Lys-[Leu⁸][des-Arg⁹] -bradykinin, DArg [Hyp³,Thi⁵,DTic⁷,Oic⁸]-bradykinin (icatibant; HOE140), 1-([2,4-dichloro-3-{([2,4-dimethylquinolin-8-yl]oxy)methyl}phenyl]sulphonyl)-N-(3-[{4-(aminomethyl)phenyl}carbonylamino)propyl)-2(S)-pyrrolidinecarboxamide (anatibant; LF160687), (E)-3-(6-acetamido-3-pyridyl)-N-(N-[2,4-dichloro-3{(2-methyl-8-quinolinyl)oxymethyl}phenyl]-N-methylaminocarbonyl-methyl)acrylamide (FR173657), [[4-[[2-[[bis(cyclohexylamino)methylene] amino]-3-(2-naphthyl)-1-oxopropyl]amino]phenyl]methyl]tributylphosphonium chloride monohydrochloride (WIN 64338), bradyzyte (British Journal of Pharmacology (2000) 129, 77 - 86), (S)-1-[4-(4-benzhydrylthiosemicarbazido)-3-nitrobenzenesulfonyl]pyrrolidine-2-carboxylic acid [2-[(2-dimethylaminoethyl)methylamino]ethyl] amide (bradyzide; (S)-4), bradykinin B(2) receptor antagonists described in Curr Med Chem. 2002 May;9(9):913-28, or mixtures thereof, or pharmaceutically acceptable salts thereof.

10. : Use according to any one of claims 1 to 9, **characterised in that** the medicament is administered as a combination medicament.

11. : Use according to any one of claims 1 to 10, **characterised in that** the medicament is administered intravenously, intraperitoneally, mucosally, especially intranasally, orally or intratracheally, or as an aerosol composition.

12. : Combination medicament comprising ACE2 and an inhibitor of the Renin-Angiotensin-System and/or a bradykinin receptor inhibitor, especially an AT₁-inhibitor, AT₂-agonist, a bradykinin receptor inhibitor, a renin inhibitor or an ACE inhibitor or mixtures of said inhibitors.

13. : Use of an inhibitor of the Renin-Angiotensin-System for the prevention or treatment of lung injuries or lung failures, preferably of severe acute lung injury induced by acid aspiration or sepsis, of lung oedemas and lung injuries and failures connected with infection with SARS coronavirus, especially of lung oedemas.

14. : Use according to claim 13 **characterised in that** said inhibitor of the Renin-Angiotensin-System is selected from the group consisting of ACE inhibitors, ACE2, AT₁-inhibitors, AT₂-receptor, AT₂-activators, renin inhibitors or combinations thereof.

15. : Use of a bradykinin receptor inhibitor for the prevention or treatment of lung injuries or lung failures, preferably of severe acute lung injury induced by acid aspiration or sepsis, of lung oedemas and lung injuries and failures connected with infection with SARS coronavirus, especially of lung oedemas.
